# EUROPÄISCHE PATENTSCHRIFT

(11) **EP 1 133 229 B1**
(45) Veröffentlichungstag und Bekanntmachung des Hinweises auf die Patenterteilung: **09.10.2002**
(21) Anmeldenummer: 99965398.3
(22) Anmeldetag: 12.11.1999
(51) Int. Cl.: A01N 43/36, C07D 207/50

(54) **FUNGIZIDE MITTEL ENTHALTEND ALS WIRKSTOFFE PYRROLIDONE**
FUNGICIDES CONTAINING PYRROLIDONES AS THEIR ACTIVE AGENTS
PRODUITS FONGICIDES CONTENANT COMME PRINCIPES ACTIFS DES PYRROLIDONES

(30) Priorität: 24.11.1998 DE 19854248
(43) Veröffentlichungstag der Anmeldung: 19.09.2001
(73) Patentinhaber: BASF AKTIENGESELLSCHAFT, 67056 Ludwigshafen (DE)
(72) Erfinder: RHEINHEIMER, Joachim, D-67063 Ludwigshafen (DE); EICKEN, Karl, D-67157 Wachenheim (DE); ROSE, Ingo, D-68159 Mannheim (DE); GROTE, Thomas, D-67105 Schifferstadt (DE); AMMERMANN, Eberhard, D-64646 Heppenheim (DE); LORENZ, Gisela, D-67434 Neustadt (DE); STRATHMANN, Siegfried, D-67117 Limburgerhof (DE); HAMPRECHT, Gerhard, D-69469 Weinheim (DE)
(86) Internationale Anmeldenummer: EP9908739
(87) Internationale Veröffentlichungsnummer: WO00030445

(56) Entgegenhaltungen:
- DE-A- 3 222 152
- M. AUGUSTIN ET AL: "Umsetzungen von 2-Arylmaleinsäureanhydriden mit Arylhydrazinen" ZEITSCHRIFT FÜR CHEMIE, Bd. 13, Nr. 6, 1973, Seiten 214-216, XP000901338 Leipzig in der Anmeldung erwähnt

## Beschreibung

Die vorliegende Erfindung betrifft neue agrochemische Zusammensetzungen mit fungizider Wirkung enthaltend Pyrrolidone als Wirkstoffe, sowie deren Verwendung bei der Behandlung von Pflanzen und in der Landwirtschaft.

Gegenstand der vorliegenden Erfindung sind Zusammensetzung enthaltend als Wirkstoffe Verbindungen der Formel I 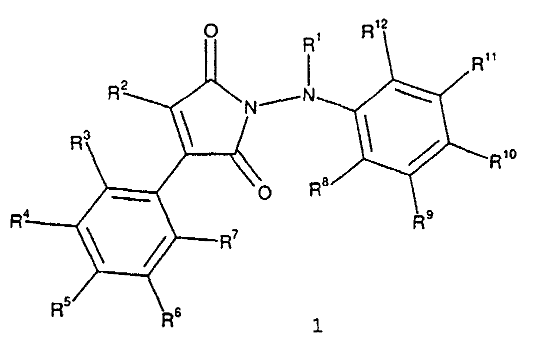 wobei die Reste folgende Bedeutungen haben:
- R¹: Wasserstoff, C₁-C₆-Alkyl, C₁-C₆-Alkylcarbonyl, Formyl oder C₁-C₆-Halogenalkylcarbonyl;
- R²: Wasserstoff, C₁-C₆-Alkyl oder C₁-C₆-Halogenalkyl;
- R³ - R¹²: Wasserstoff, Halogen, C₁-C₈-Cycloalkyl, C₁-C₆-Alkyl, C₁-C₆-Halogenalkyl, C₁-C₆-Alkoxy, C₁-C₆-Halogenalkoxy, C₁-C₆-Alkylsulfonyl, C₁-C₆-Halogenalkylsulfonyl, Formyl, C₁-C₆-Alkylcarbonyl, Cyano, C₁-C₆-Alkylthio oder Phenyl, welches ggf. durch Halogenatome, C₁-C₆-Alkyl- oder C₁-C₆-Halogenalkyl-Gruppen substituiert sein kann, sowie deren landwirtschaftlich einsetzbaren Salze.

Einige der Verbindungen der Formel I sind literaturbekannt (DE-A-3 222 152, M. Augustin und P. Reinemann, Z. Chem. Band 13, S. 214.216 (1973) bzw. sind kommerziell erhältlich. Hierbei handelt es sich um die folgenden Verbindungen:
1-Anilino-3-phenyl-pyrrol-2,5-dion,
1-Anilino-3-p-tolyl-pyrrol-2,5-dion,
1-(N-Methyl-anilino)-3-p-tolyl-pyrrol-2,5-dion,
1-Anilino-3-(3-chloro-phenyl)-pyrrol-2,5-dion,
1-Anilino-3-(4-chloro-phenyl)-pyrrol-2,5-dion,
1-Anilino-3-(4-bromo-phenyl)-pyrrol-2,5-dion,
3-(4-Chloro-phenyl)-1-(N-methyl-anilino)-pyrrol-2,5-dion,
1-(4-Chloro-anilino)-3-p-tolyl-pyrrol-2,5-dion,
3-(4-Bromo-phenyl)-1-(4-methyl-anilino)-pyrrol-2,5-dion,
1-(4-Chloro-anilino)-3-(4-chloro-phenyl)-pyrrol-2,5-dion,
1-Anilino-3-(3, 4-dichlor-phenyl)-pyrrol-2,5-dion,
3-(4-Bromo-phenyl)-1-(4-methoxy-anilino)-pyrrol-2,5-dion,
3-(4-Chlor-phenyl)-1-(3,4-dichlor-anilino)-pyrrol-2,5-dion,
3-(4-Methoxy-phenyl)-1-(N-methyl-anilino)-pyrrol-2,5-dion,
1-Anilino-3-(4-methoxy-phenyl)-pyrrol-2,5-dion,
1-(4-Chloro-anilino)-3-(4-methoxy-phenyl)-pyrrol-2,5-dion,
1-(4-Methoxy-anilino)-3-(4-methoxy-phenyl)-pyrrol-2,5-dion,
1-(2-Methoxy-anilino)-3-(4-methoxy-phenyl)-pyrrol-2,5-dion,
1-(4-Fluoro-anilino)-3-(4-chloro-phenyl)-pyrrol-2,5-dion,
3-(4-Chlor-phenyl)-1-(4-methyl-anilino)-pyrrol-2,5-dion,
1-(4-Methyl-anilino)-3-phenyl-pyrrol-2,5-dion,
3-(4-Chlor-phenyl)-1-(2, 4-dichlor-anilino)-pyrrol-2,5-dion,
1-(2, 4-Dichlor-anilino)-3-phenyl-pyrrol-2,5-dion,
1-(2, 4-Dichlor-anilino)-3-(4-methoxy-phenyl)-pyrrol-2,5-dion.

Eine fungizide Wirkung dieser Verbindungen ist bisher noch nicht beschrieben.

Überraschenderweise wurde gefunden, daß Verbindungen der Formel I eine fungizide Wirkung aufweisen. Sie eignen sich zur Bekämpfung von Schadpilzen bei der Behandlung von Pflanzen, als auch zur therapeutischen Behandlung von durch Schadpilzen verursachten Erkrankungen am Menschen, sowie zur veterinärischen Behandlung bei Säugetieren.

Verbindungen der Formel I lassen sich analog zu dem in der Literatur (Z. Chem. Band 13, S. 214.216 (1973)) beschriebenen Verfahren herstellen. Die Ausgangsstoffe sind entweder literaturbekannt oder kommerziell erhältlich.

Bei der Definition der Substituenten R¹ bis R¹² stehen die angegebenen Begriffe als Sammelbegriff für eine Gruppe von Verbindungen.

Halogen steht jeweils für Fluor, Brom, Chlor oder Iod, insbesondere für Fluor oder Chlor.

Ferner stehen beispielsweise:
- C₁-C₆-Alkyl für: Methyl, Ethyl, n-Propyl, 1-Methylethyl, n-Butyl, 1-Methylpropyl, 2-Methylpropyl oder 1,1-Dimethylethyl, insbesondere für Ethyl;
- C₁-C₆-Halogenalkyl für: einen C₁-C₆-Alkylrest wie vorstehend genannt, der partiell oder vollständig durch Fluor, Chlor, Brom und/oder Iod substituiert ist, z.B. Trichlormethyl, Trifluormethyl, 2-Fluorethyl, 2-Chlorethyl, 2-Bromethyl, 2,2-Difluorethyl, 2,2,2-Trifluorethyl, 2,2,2-Trichlorethyl, 2-Fluorpropyl, 3-Fluorpropyl, 2-Chlorpropyl oder 3-Chlorpropyl, insbesondere für 2-Fluorethyl oder 2-Chlorethyl;
- C₁-C₆-Alkoxy für: Methoxy, Ethoxy, n-Propoxy, 1-Methylethoxy, n-Butoxy, 1-Methylpropoxy, 2-Methylpropoxy oder 1,1-Dimethylethoxy, insbesondere für Methoxy oder Ethoxy;
- C₃-C₈-Cycloalkyl für: z.B. Cyclopropyl, Cyclobutyl, Cyclopentyl, Cyclohexyl, Cycloheptyl, Cyclooctyl;
- Halogen-C₁-C₆-Alkoxy für: einen C₁-C₆-Alkylrest wie vorstehend, der durch Fluor, Chlor oder Brom substituiert ist;
- C₁-C₆-Alkyl-carbonyl für: einen C₁-C₆-Alkylrest wie vorstehend, wie z.B. Acetyl, Propionyl, Butyryl;
- Halogen-C₁-C₆-Alkyl-carbonyl für: einen C₁-C₆-Alkyl-carbonylrest wie vorstehend, der durch Fluor, Chlor oder Brom substituiert ist;
- C₁-C₆-Alkylsulfonyl für: eine Sulfonylgruppe, die durch einen C₁-C₆-Alkylrest wie vorstehend genannt substituiert ist;
- Halogen- C₁-C₆-Alkylsulfonyl für: einen C₁-C₆-Alkylsulfonylrest wie vorstehend genannt, der durch Fluor, Chlor oder Brom substituiert ist;
- C₁-C₆-Alkylthio für: ein Schwefelatom, das durch einen C₁-C₆-Alkylrest wie vorstehend genannt substituiert ist;
- ein gegebenenfalls substituierter Phenylrest: ein Phenylrest, der ein- oder mehrfach substituiert ist. Die Substituenten sind beliebig, beispielsweise folgende: Halogenatome, C₁-C₆-Alkyl oder Halogen-C₁-C₆-Alkyl.

Folgende in den Punkten 1. - 4, genannten Verbindungen kommen im Sinne der vorliegenden Erfindung bevorzugt im Hinblick auf die genannten Substituentendefinitionen, jeweils für sich alleine oder in Kombination miteinander, in Frage:
1. Verbindungen der Formel I, wobei R¹ die folgenden Bedeutungen hat: Wasserstoff, Methyl, Ethyl oder Formyl, insbesondere Wasserstoff oder Methyl.
2. Verbindungen nach Punkt 1, wobei R² die folgenden Bedeutungen hat: Wasserstoff, Methyl, Ethyl, Trifluormethyl, insbesondere Wasserstoff.
3. Verbindungen nach den Punkten 1 oder 2, wobei R³-R¹² die folgenden Bedeutungen haben: Wasserstoff, Fluor, Chlor, Methyl, Ethyl, Propyl, Butyl, Trifluormethyl, Difluormethyl, Trifluormethoxy, Difluormethoxy, Methoxy, Methylthio, Cyano.
4. Verbindungen nach den Punkten 1 bis 3, wobei mindestens zwei der Reste R⁸-R¹² sowie außerdem mindestens zwei der Reste R³-R⁷ Wasserstoff bedeuten und die übrigen für Wasserstoff, Fluor, Chlor, Methyl, Ethyl, Propyl, Butyl, Trifluormethyl, Trifluormethoxy oder Difluormethoxy stehen.

Die beiden Phenylringe sind bevorzugt unsubstituiert (R³- R¹² = H) oder bevorzugt ein-, zwei- oder dreifach substituiert, wobei vorwiegend folgende Substituenten in Frage kommen: C₁-C₆-Alkyl, Halogen, Halogen-C₁-C₆-Alkyl, Halogen-C₁-C₆-Alkoxy, insbesondere Methyl, iso-Propyl, Fluor, Chlor, Trifluormethyl oder Trifluormethoxy.

Die zuvor genannten Verbindungen haben sich als in der Regel besonders wirksam erwiesen.

Im Sinne der vorliegenden Erfindung kommen beispielsweise folgende Verbindungen in Tabelle 1 als fungizide Wirkstoffe in Frage:

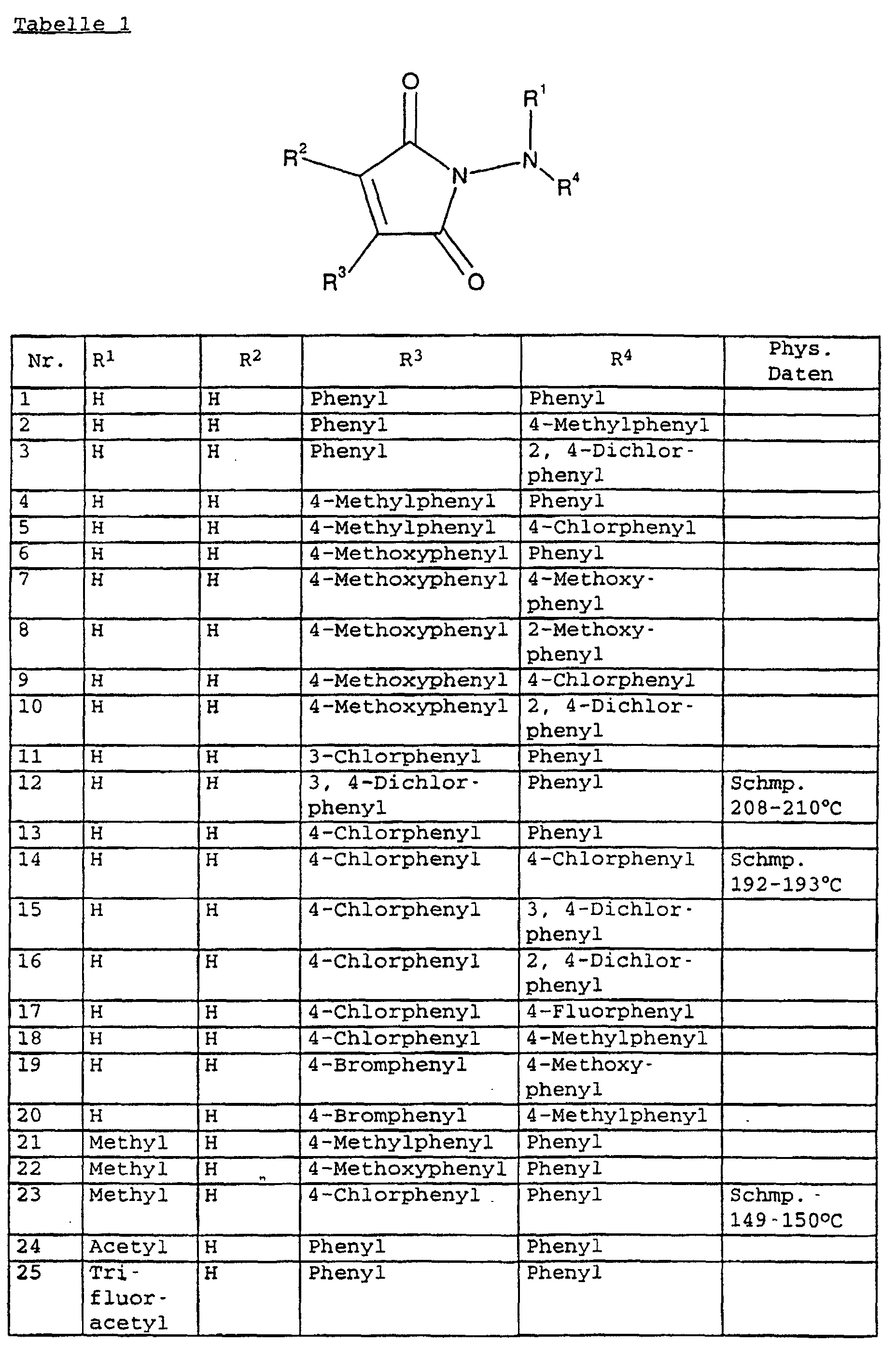

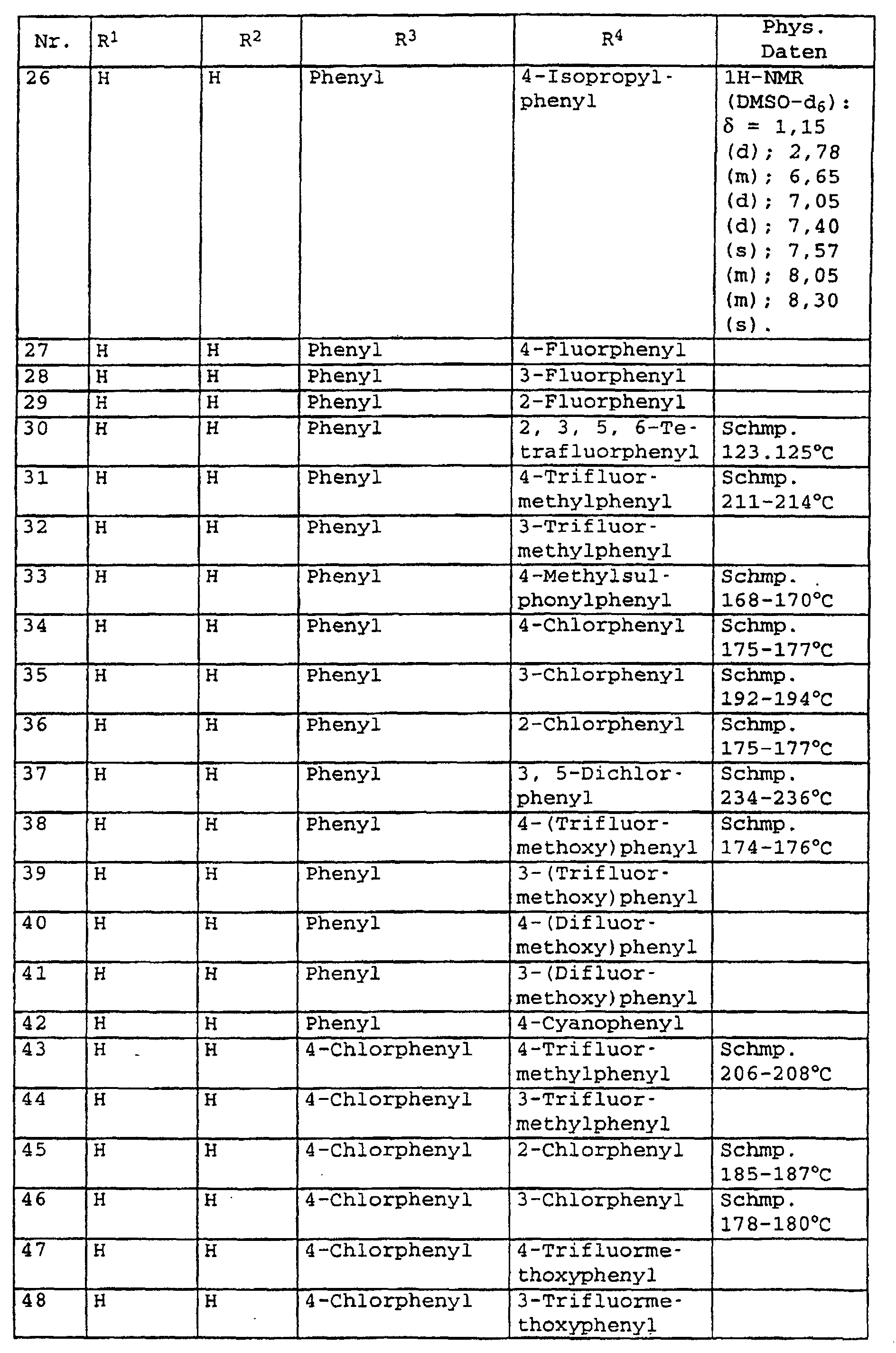

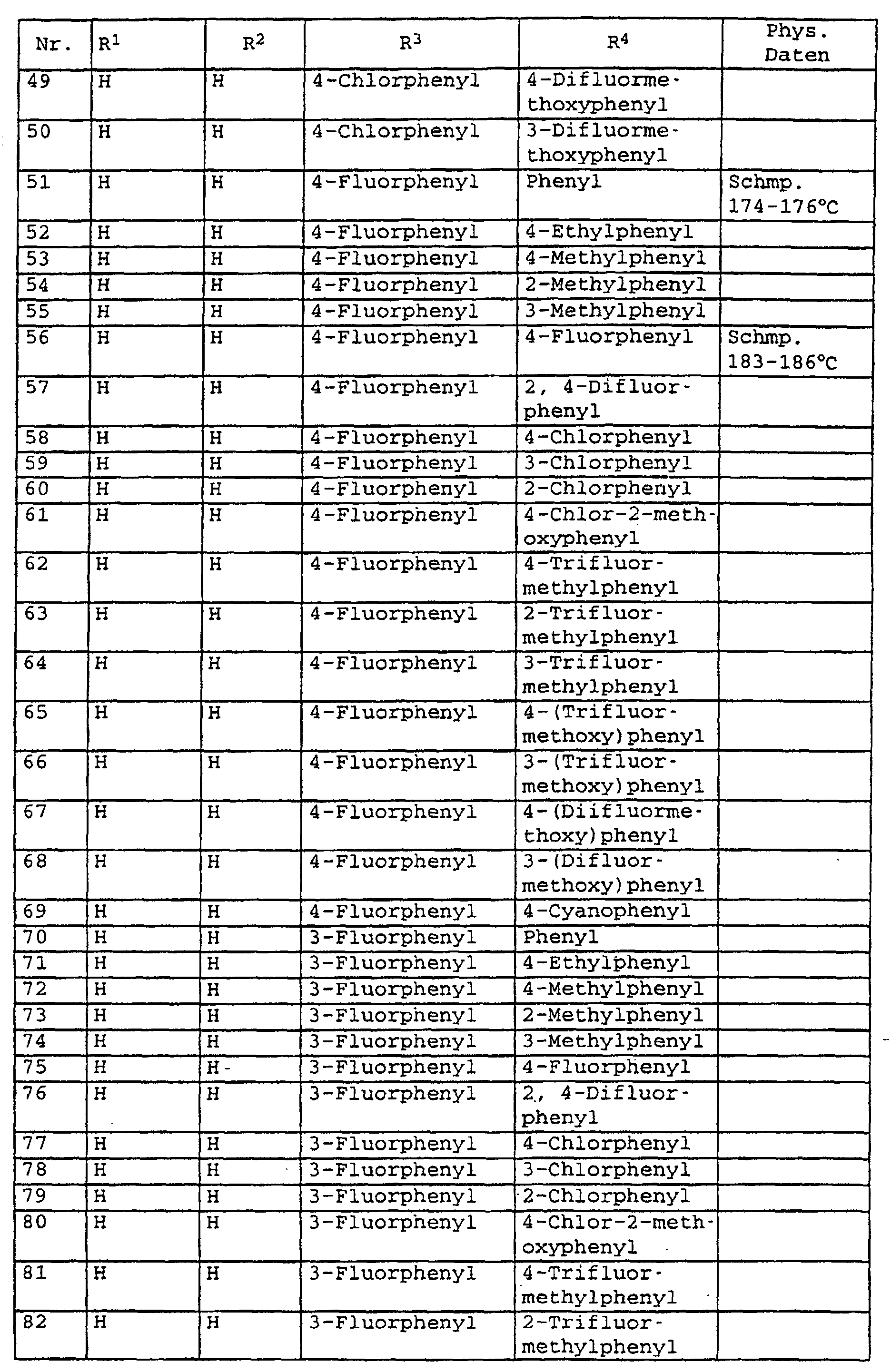

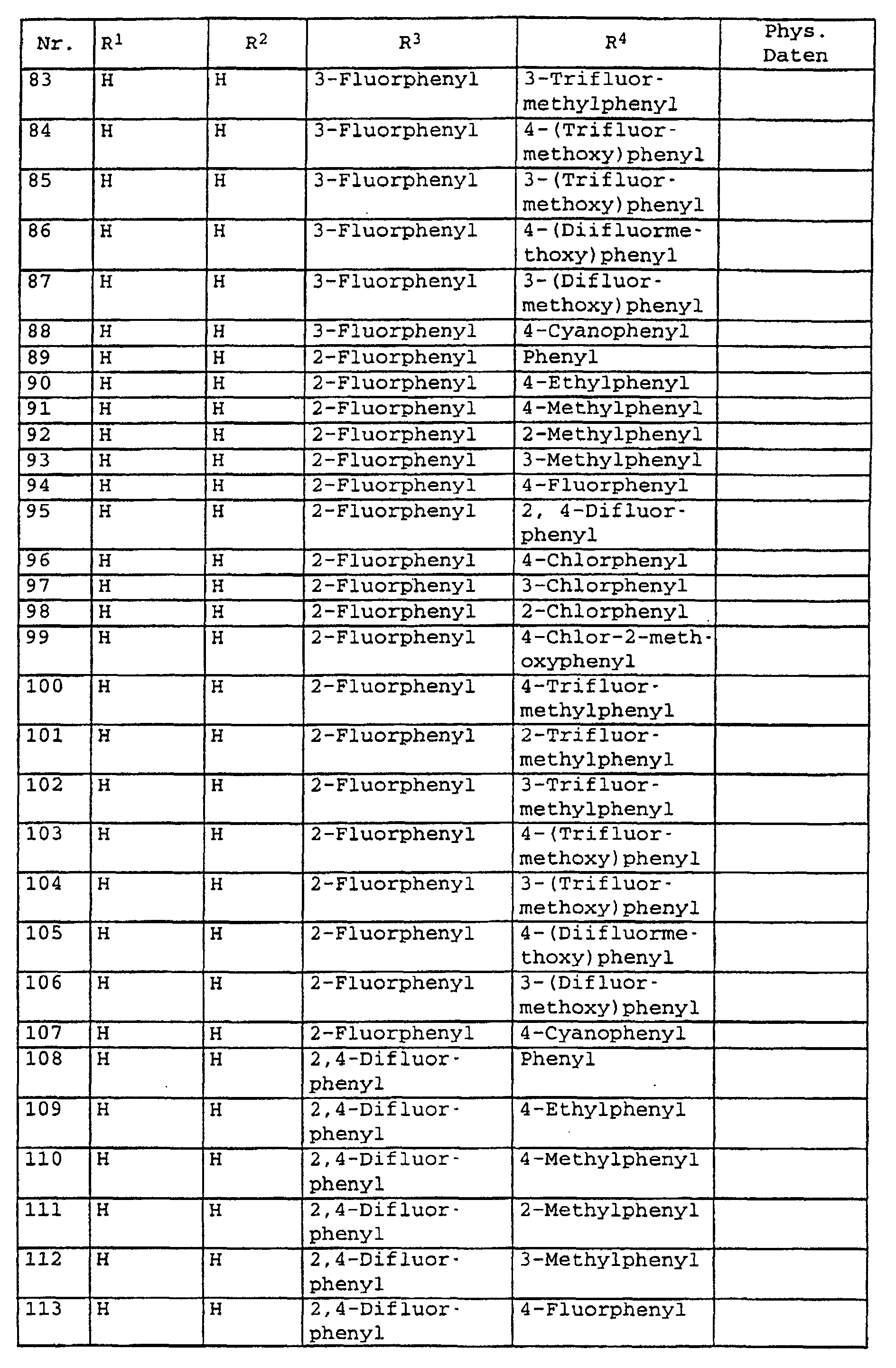

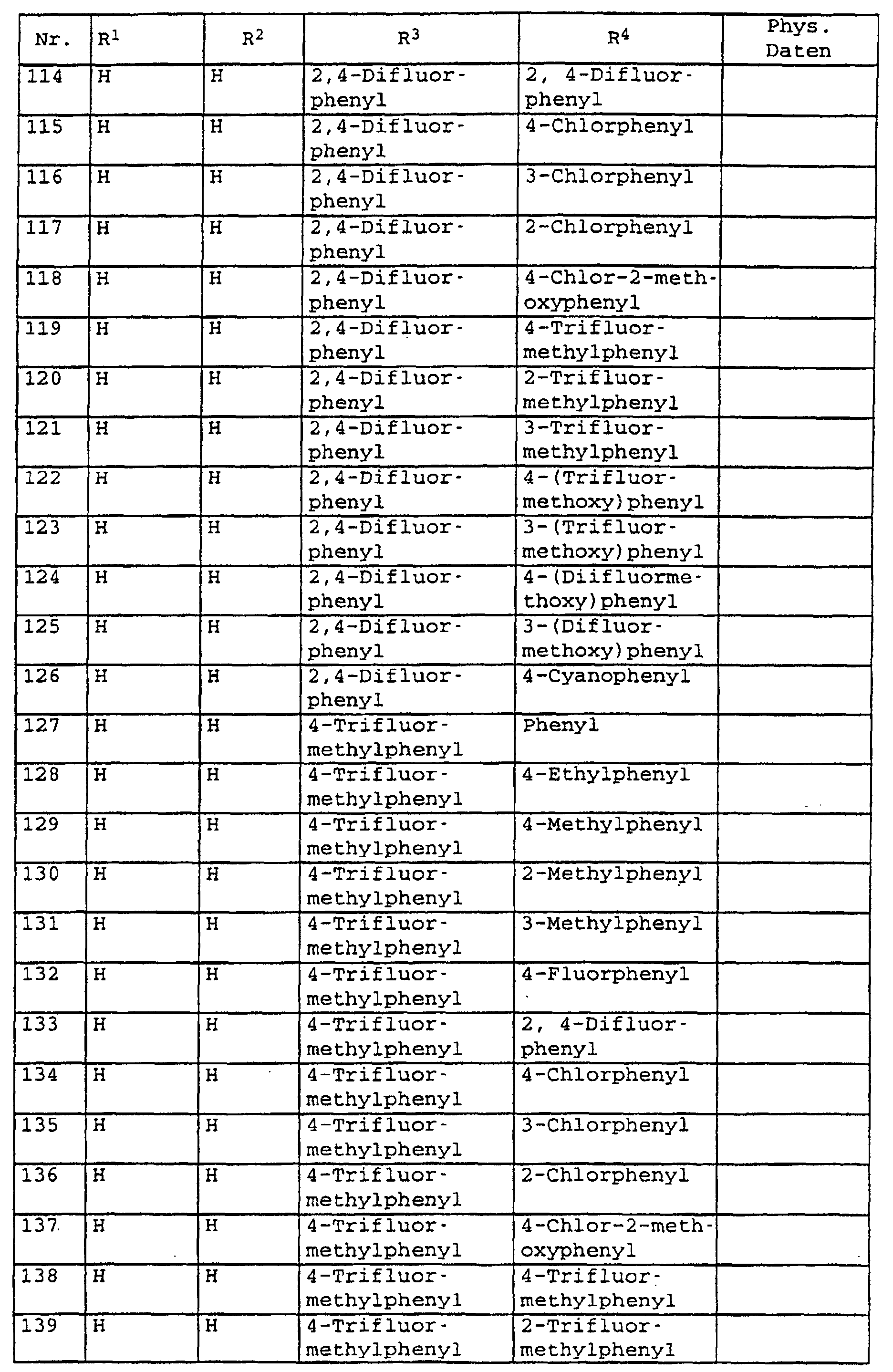

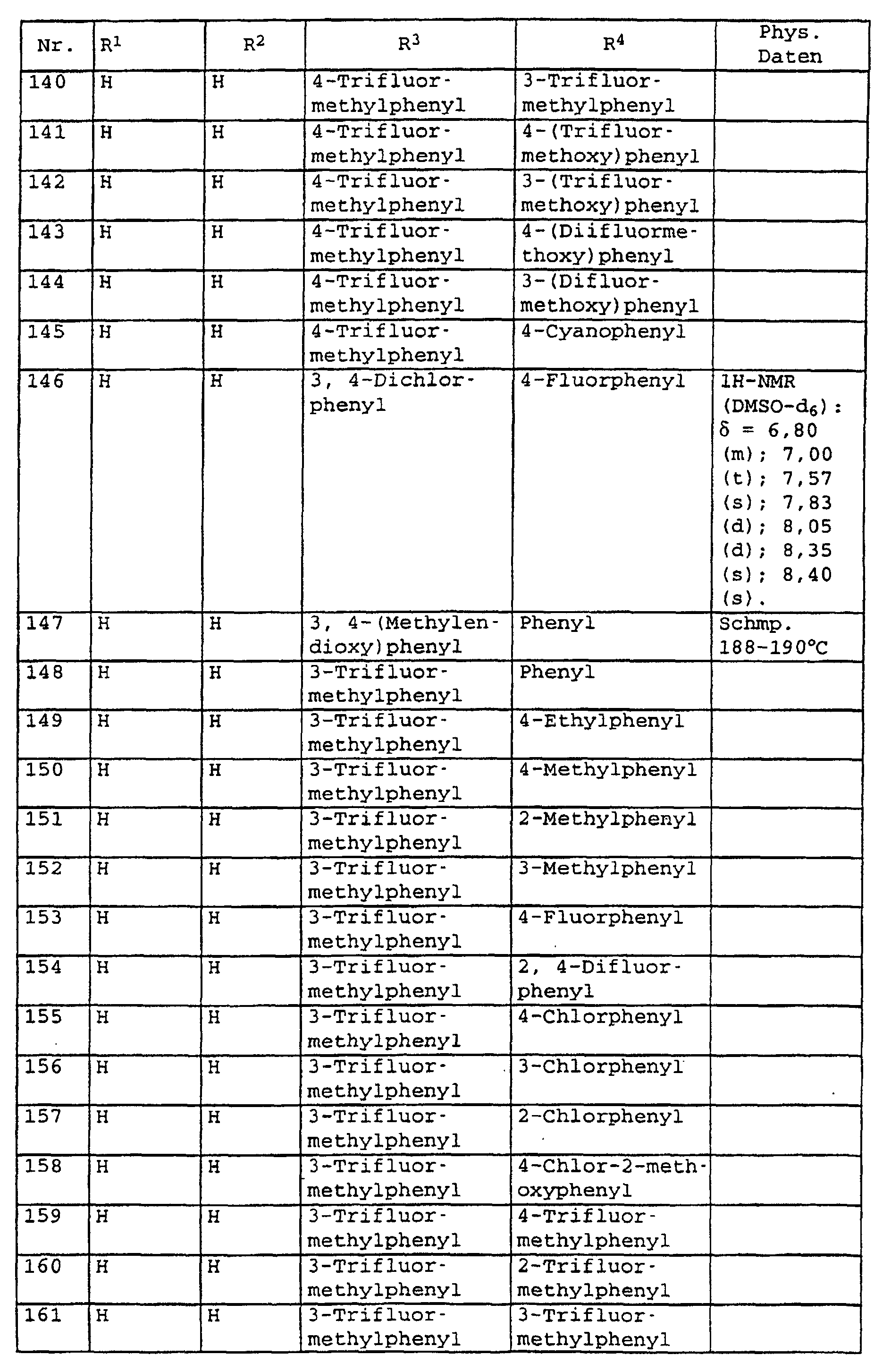

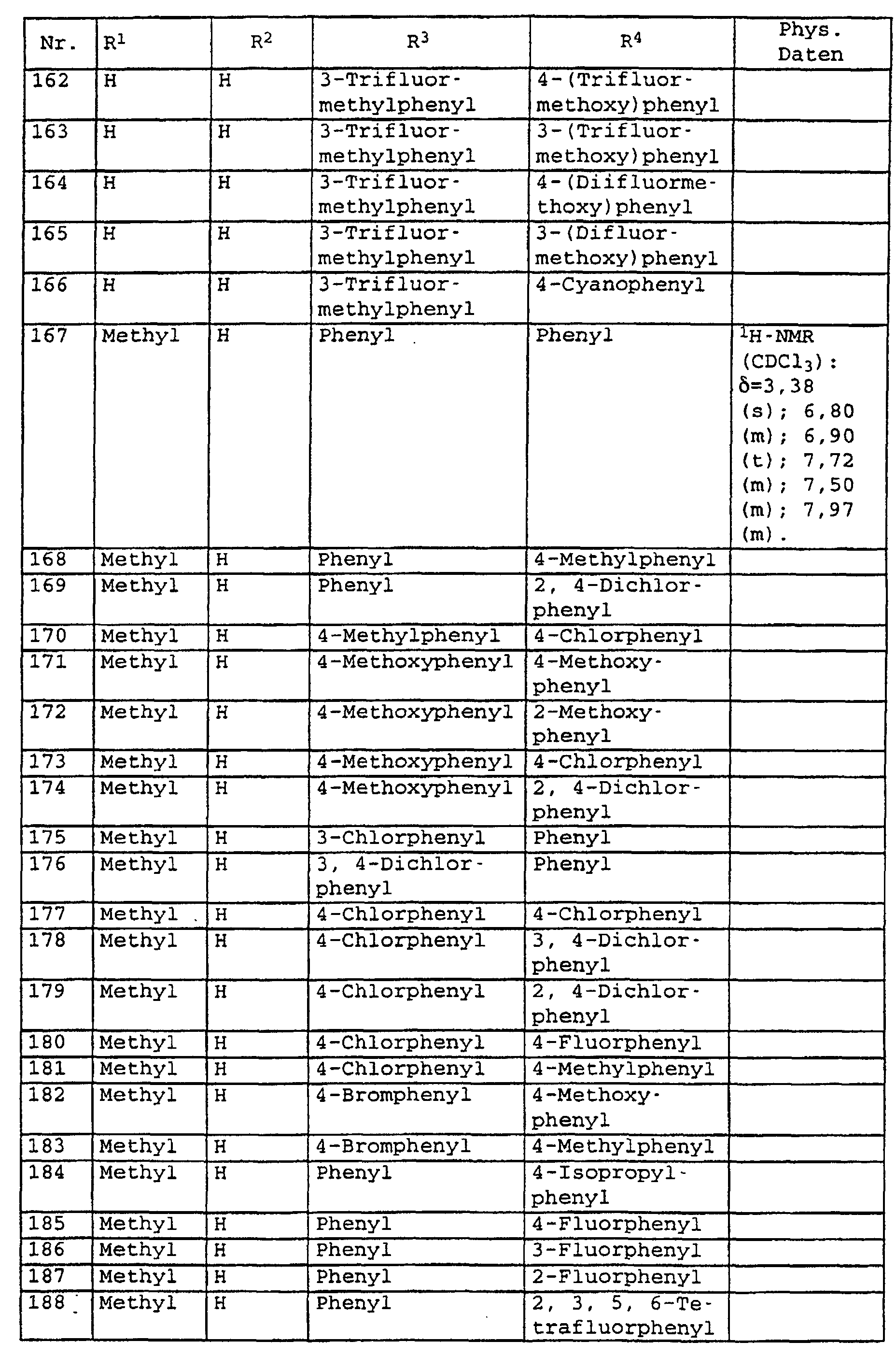

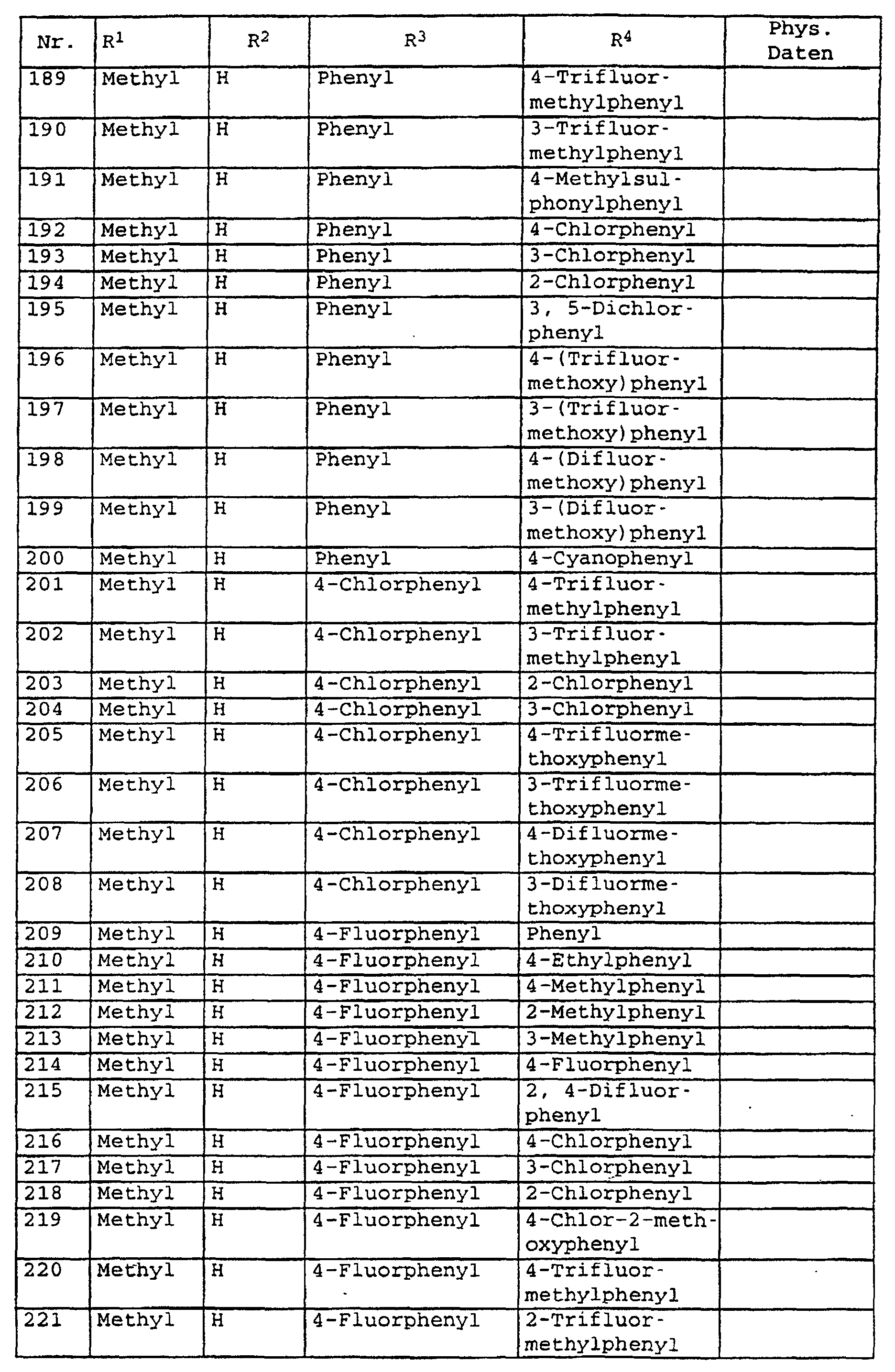

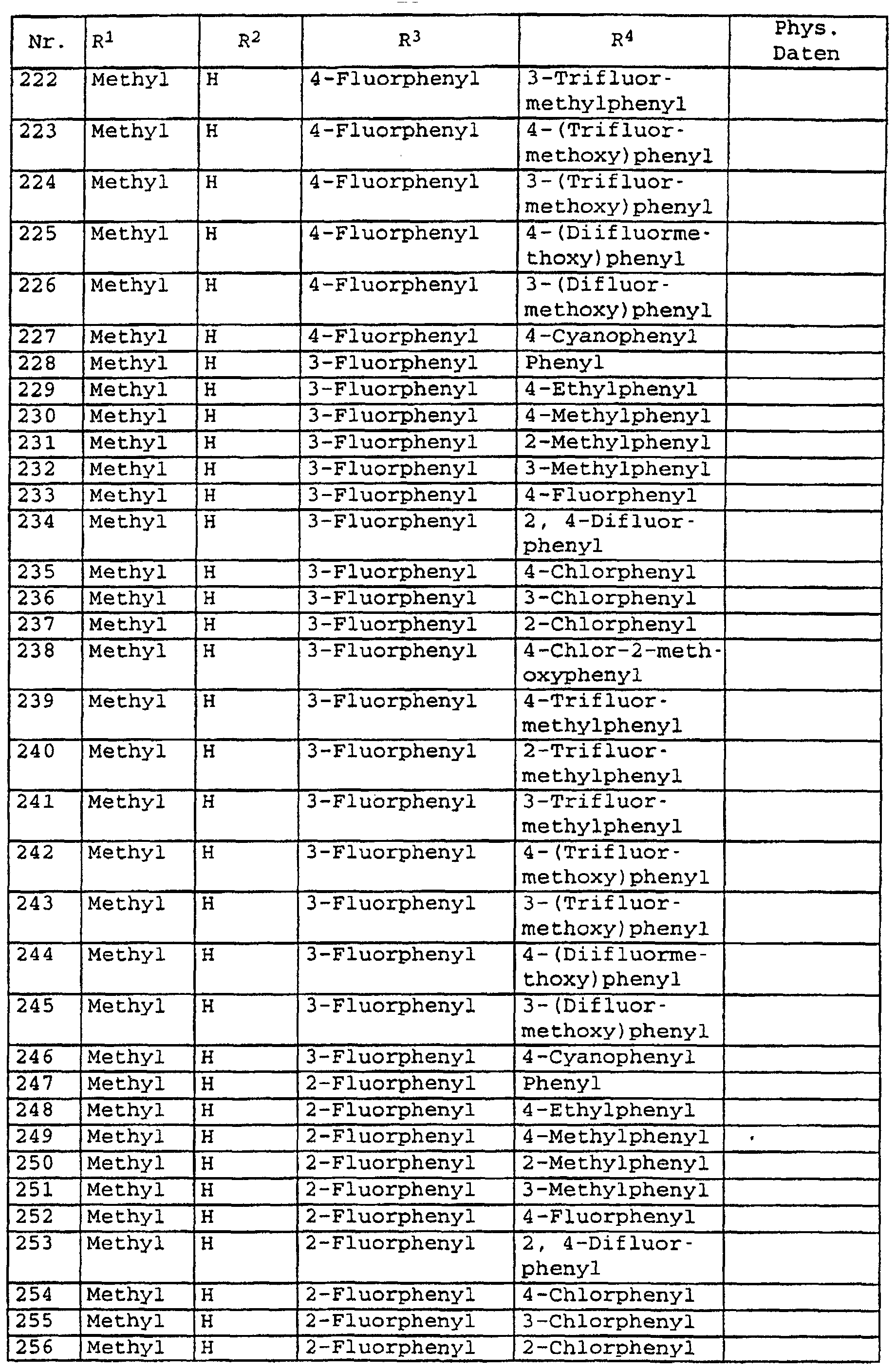

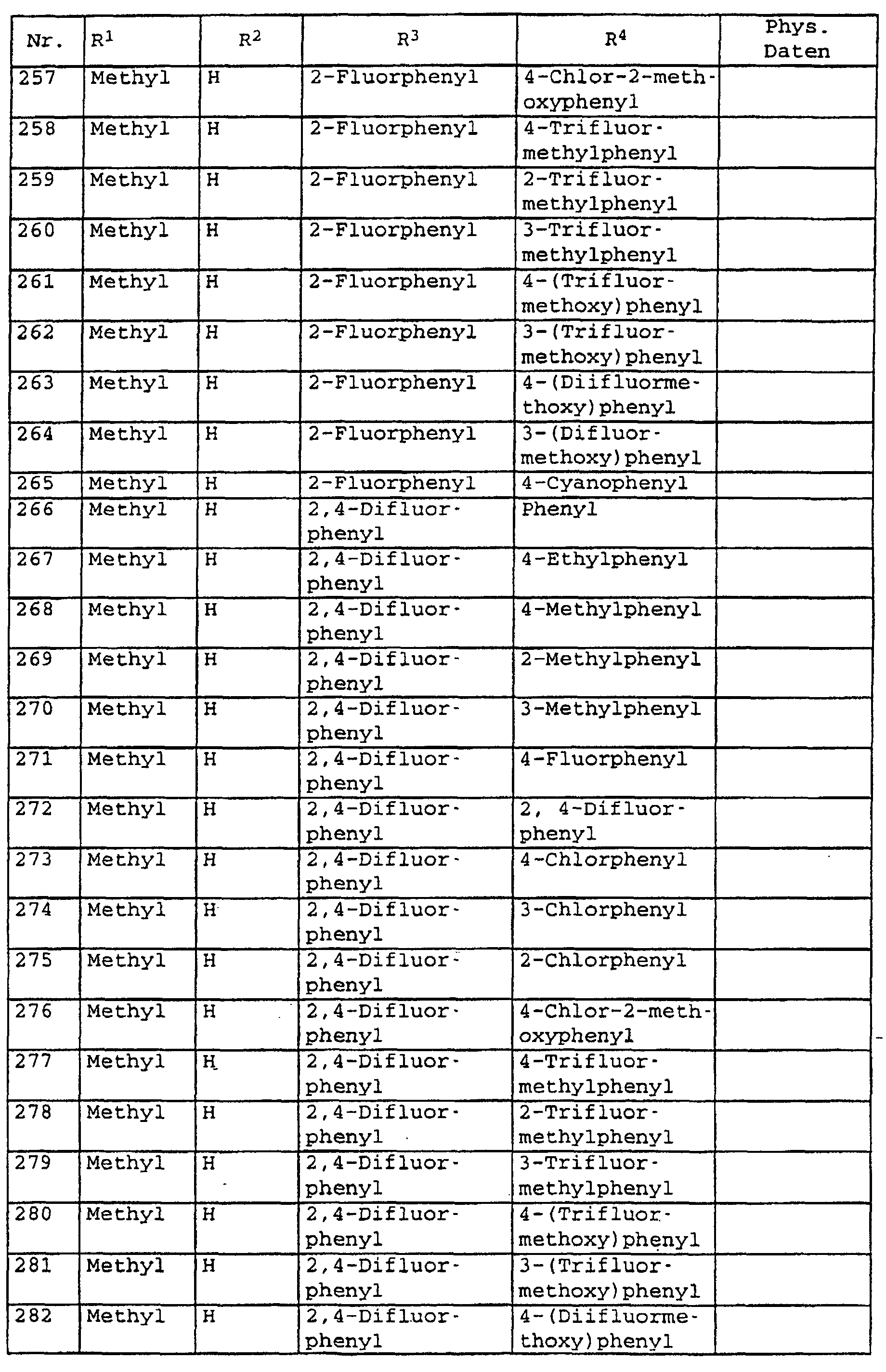

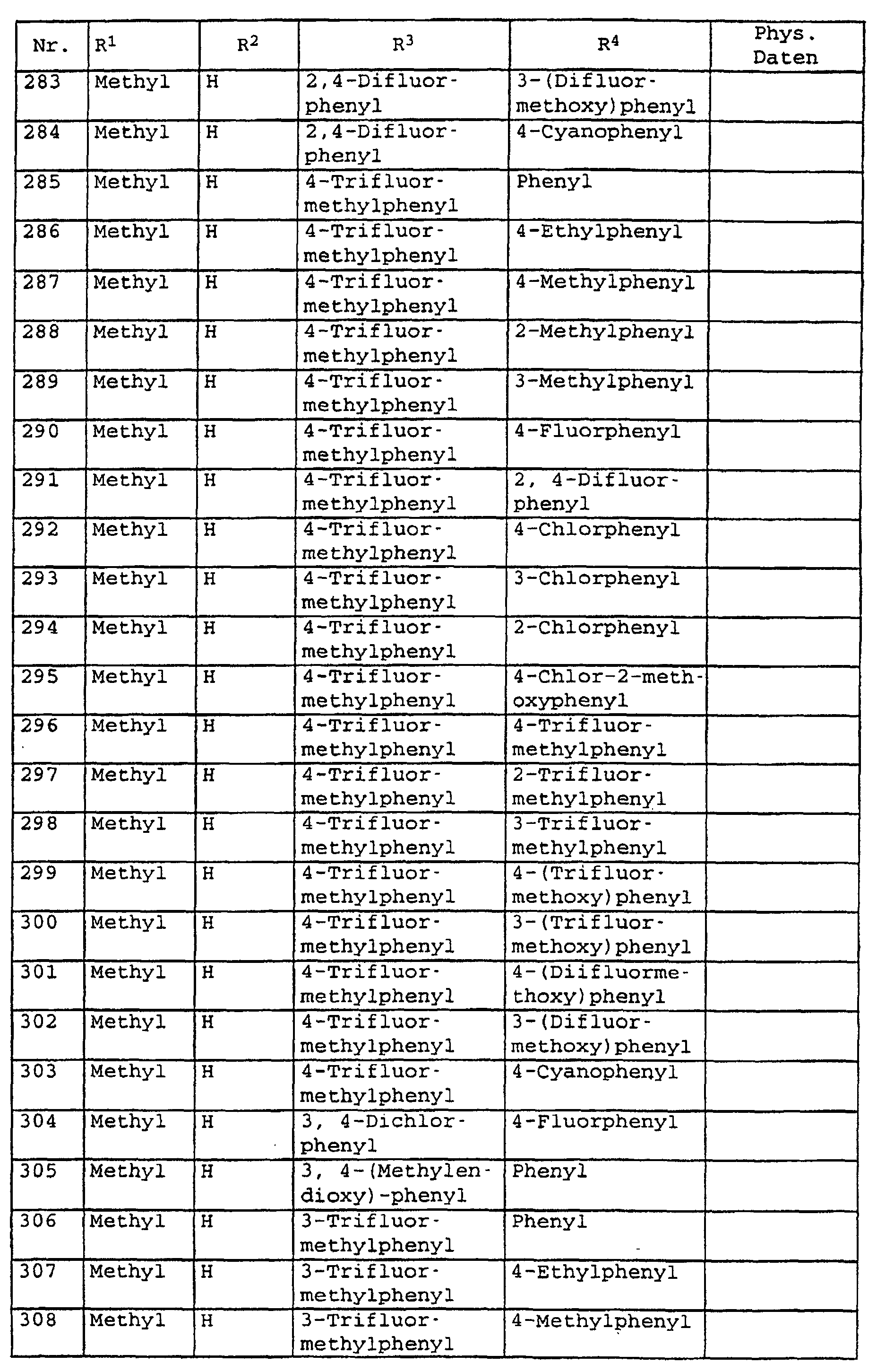

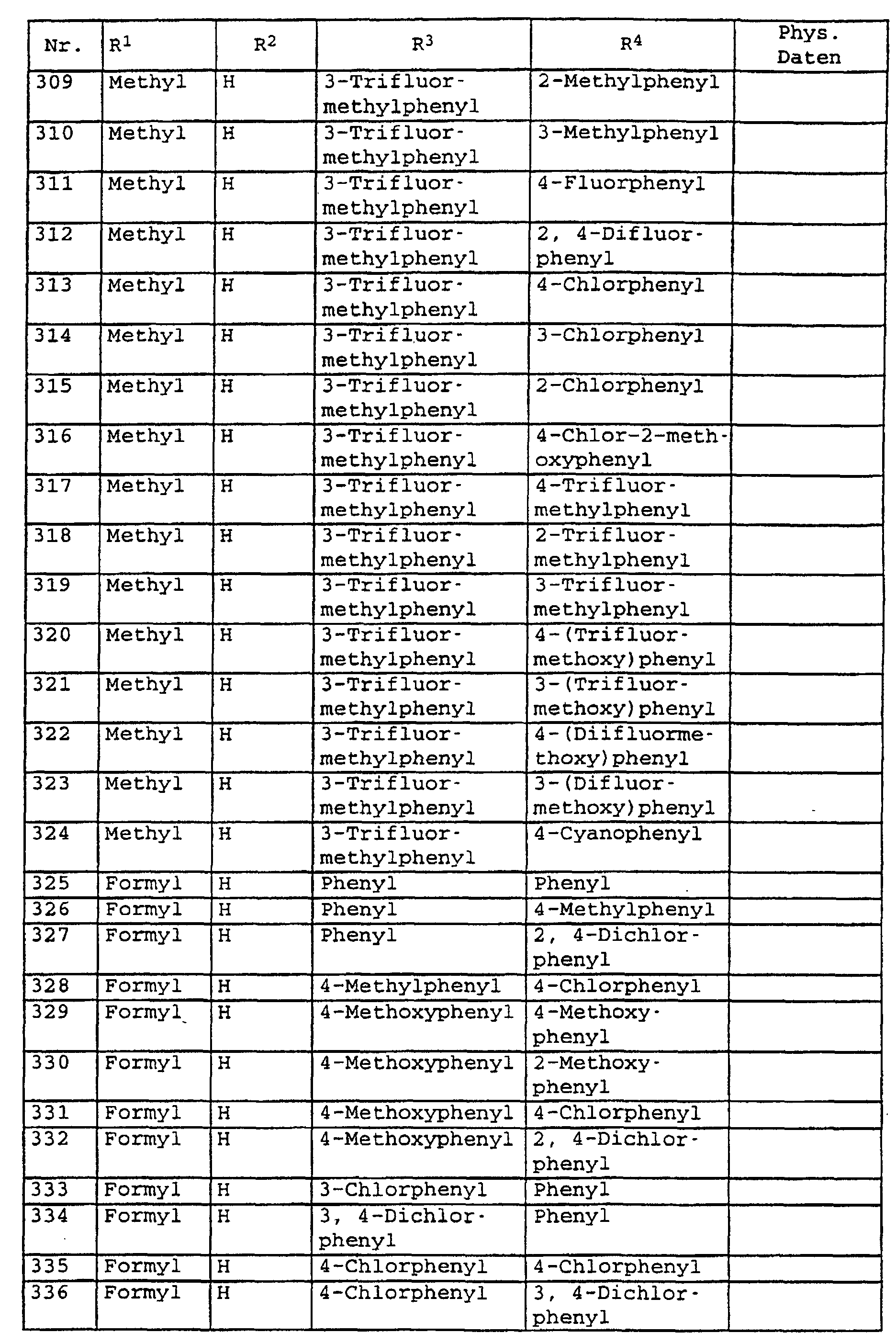

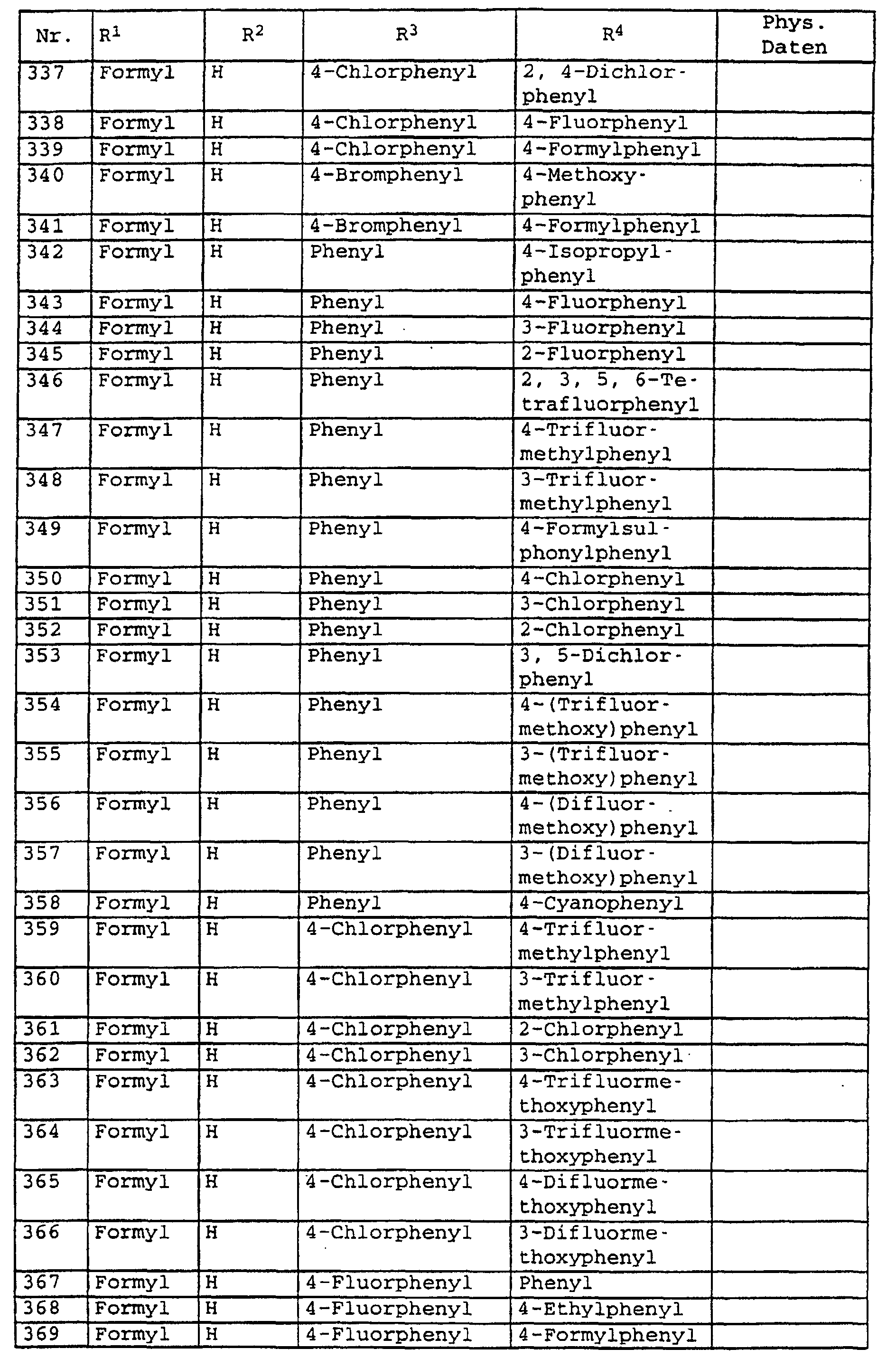

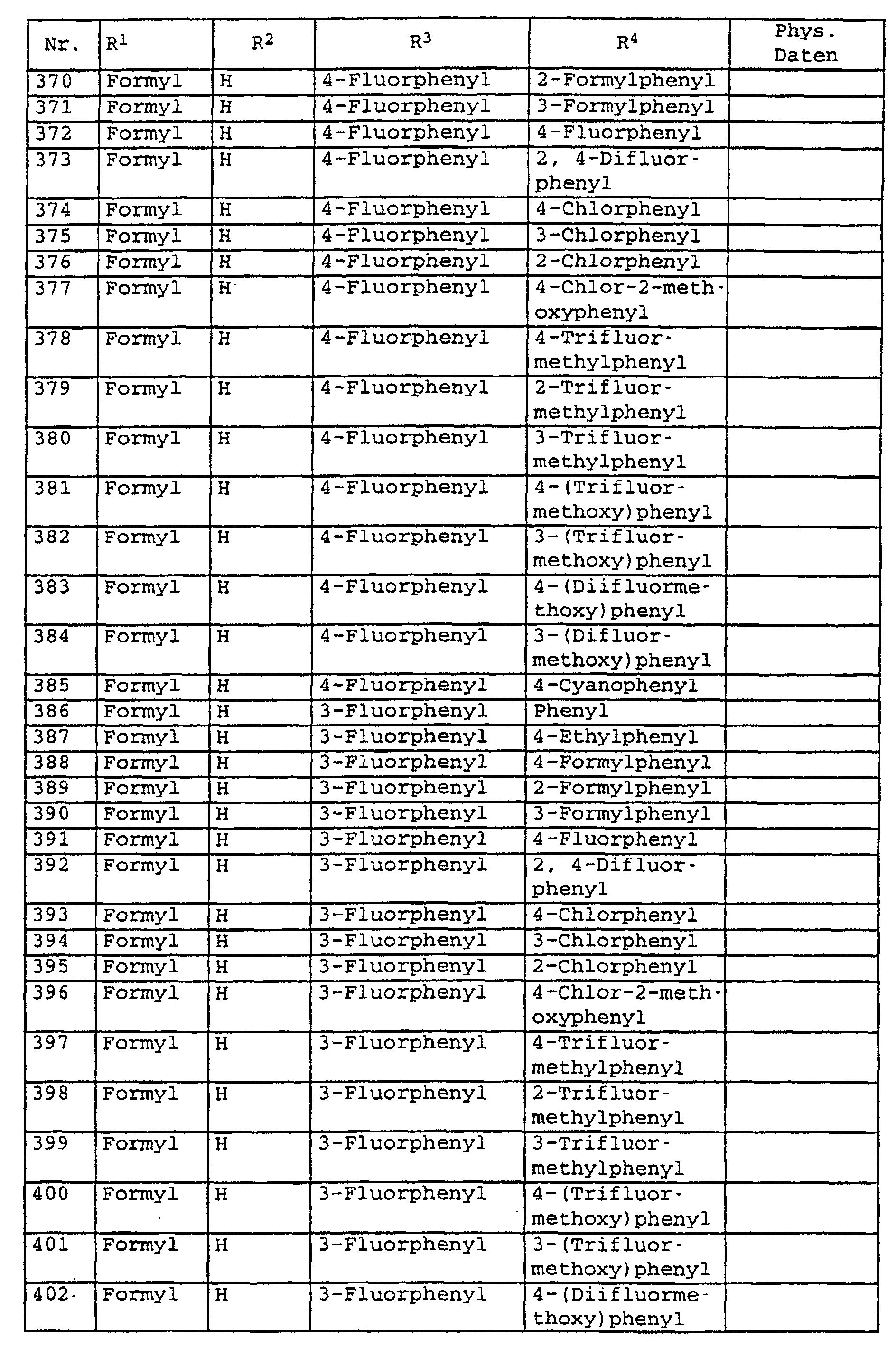

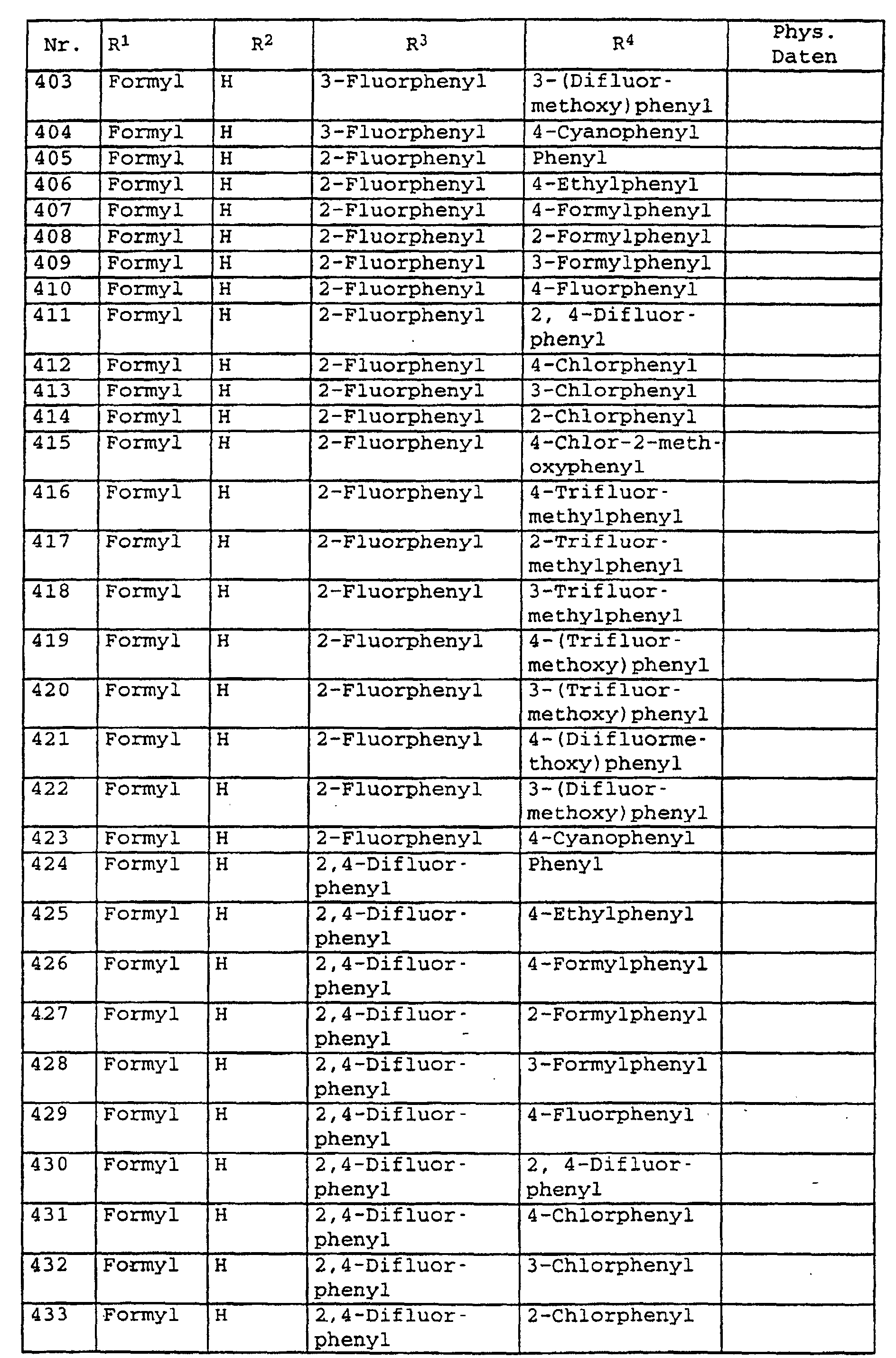

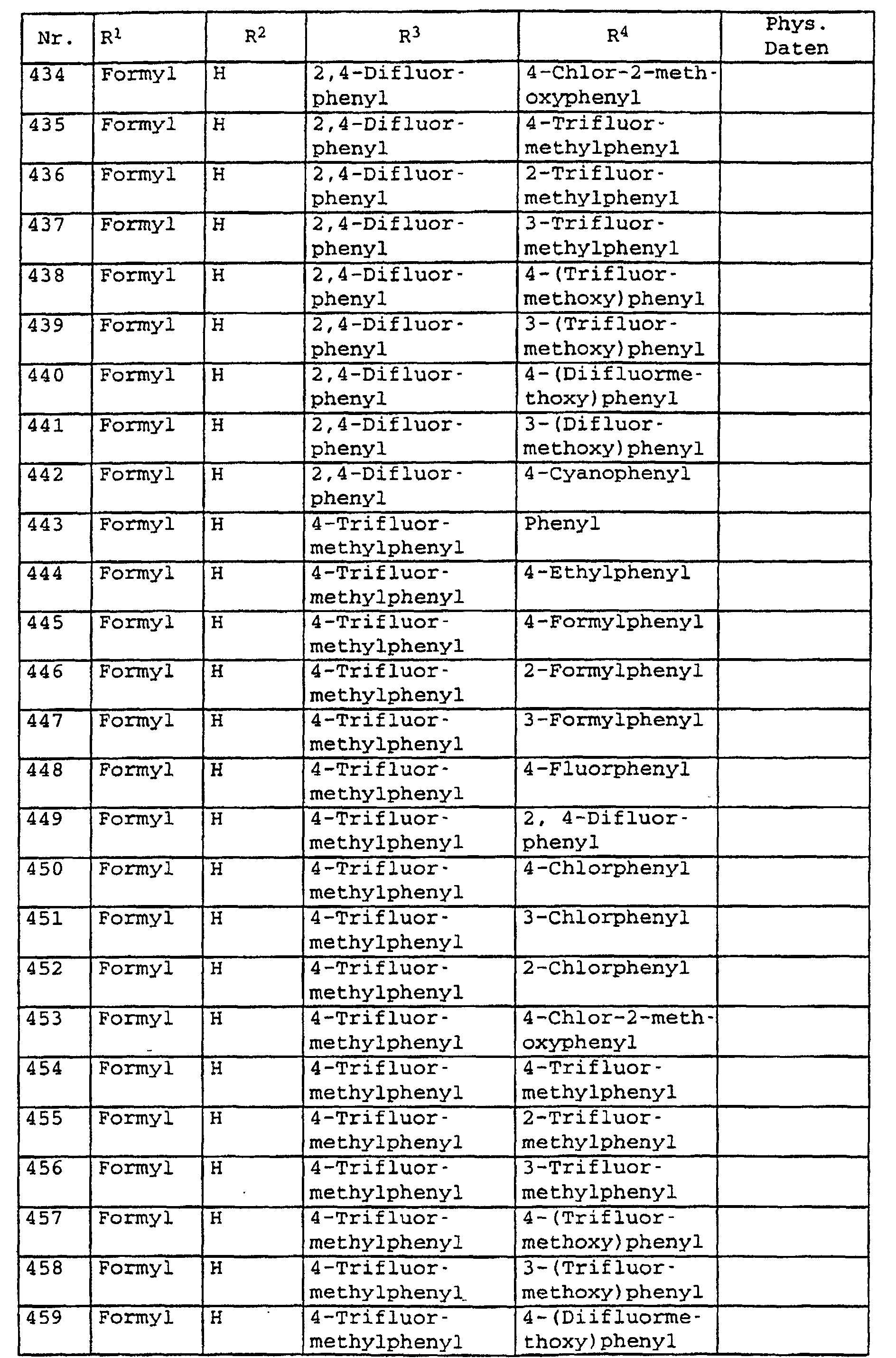

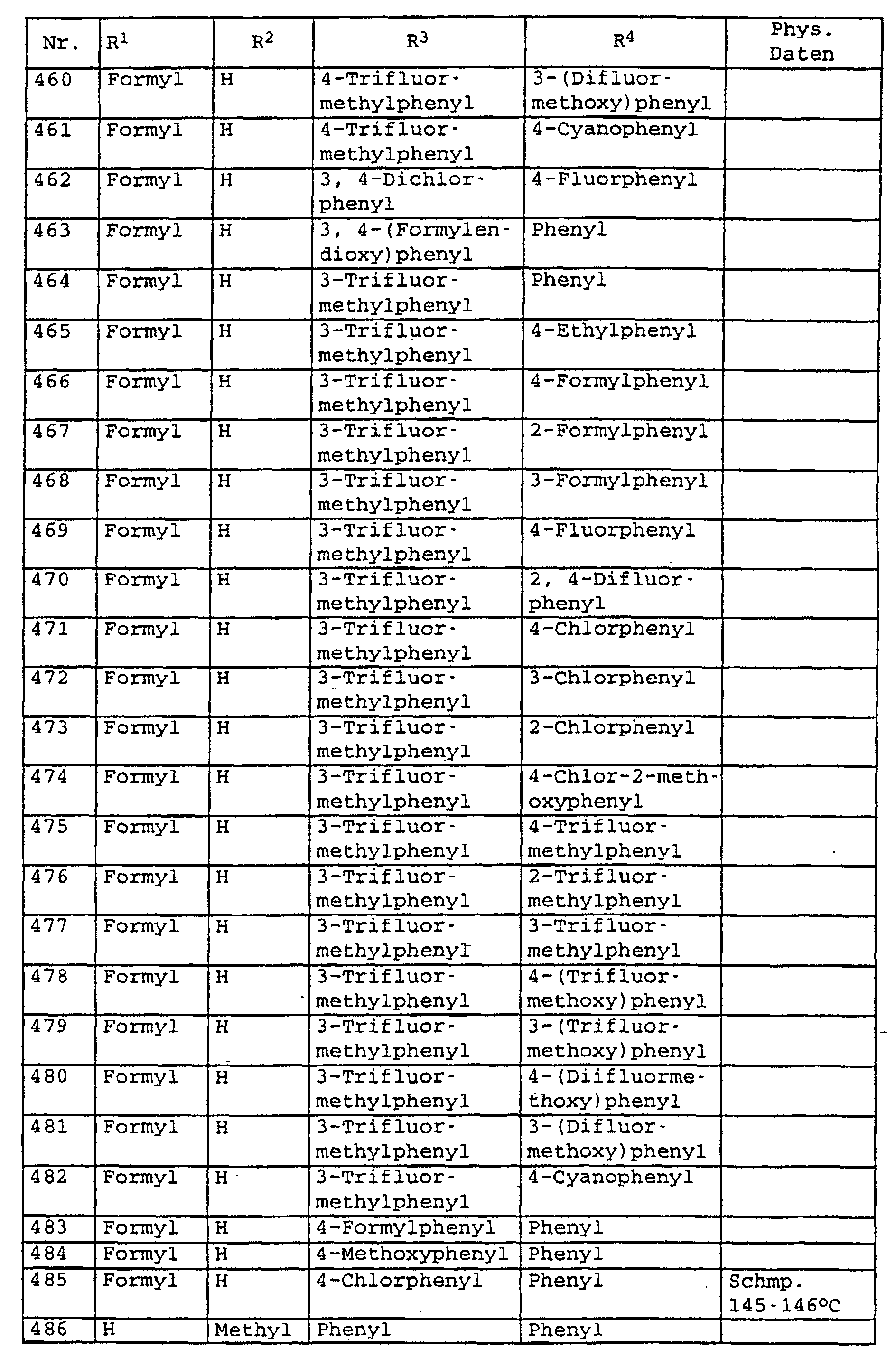

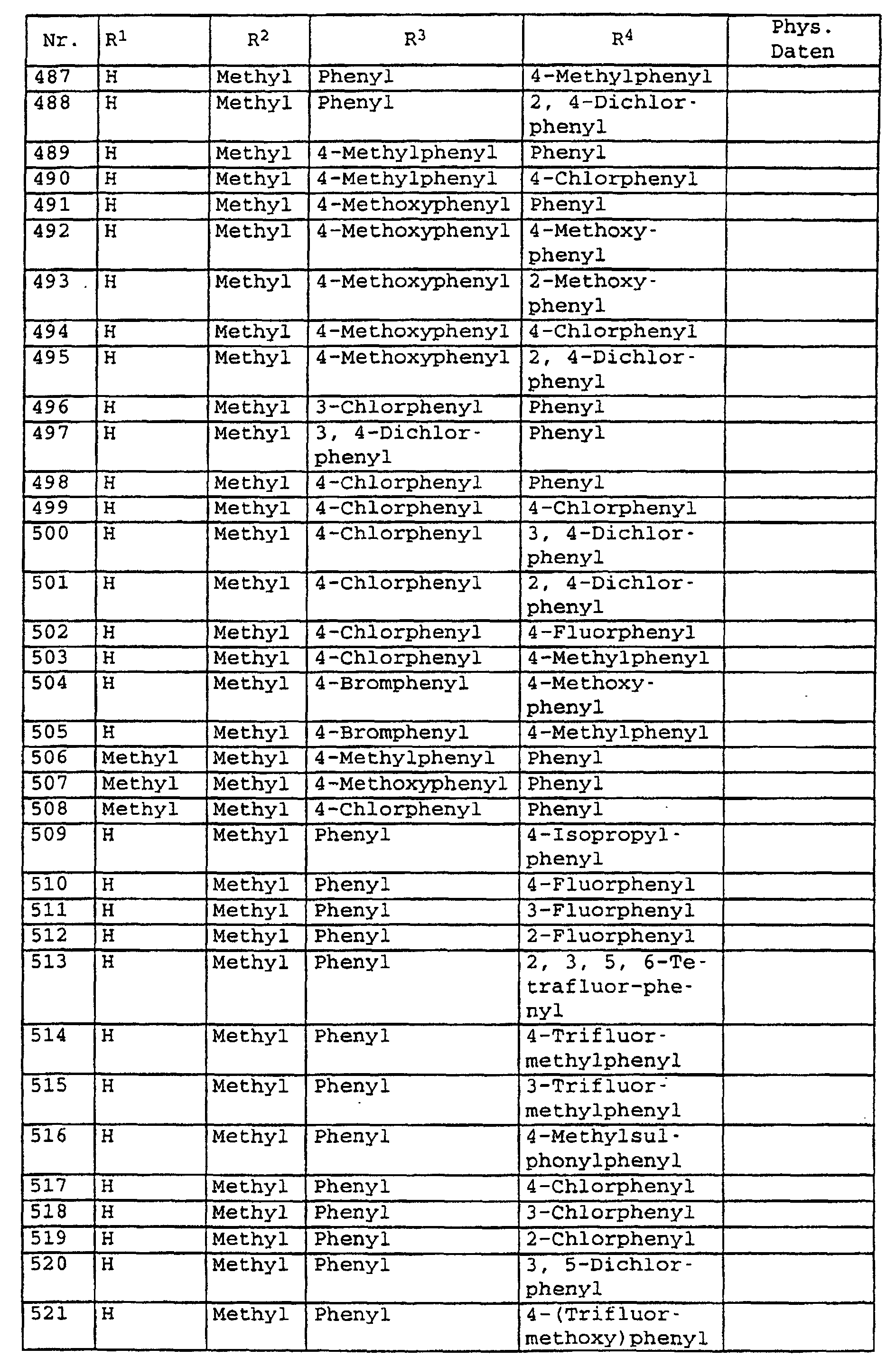

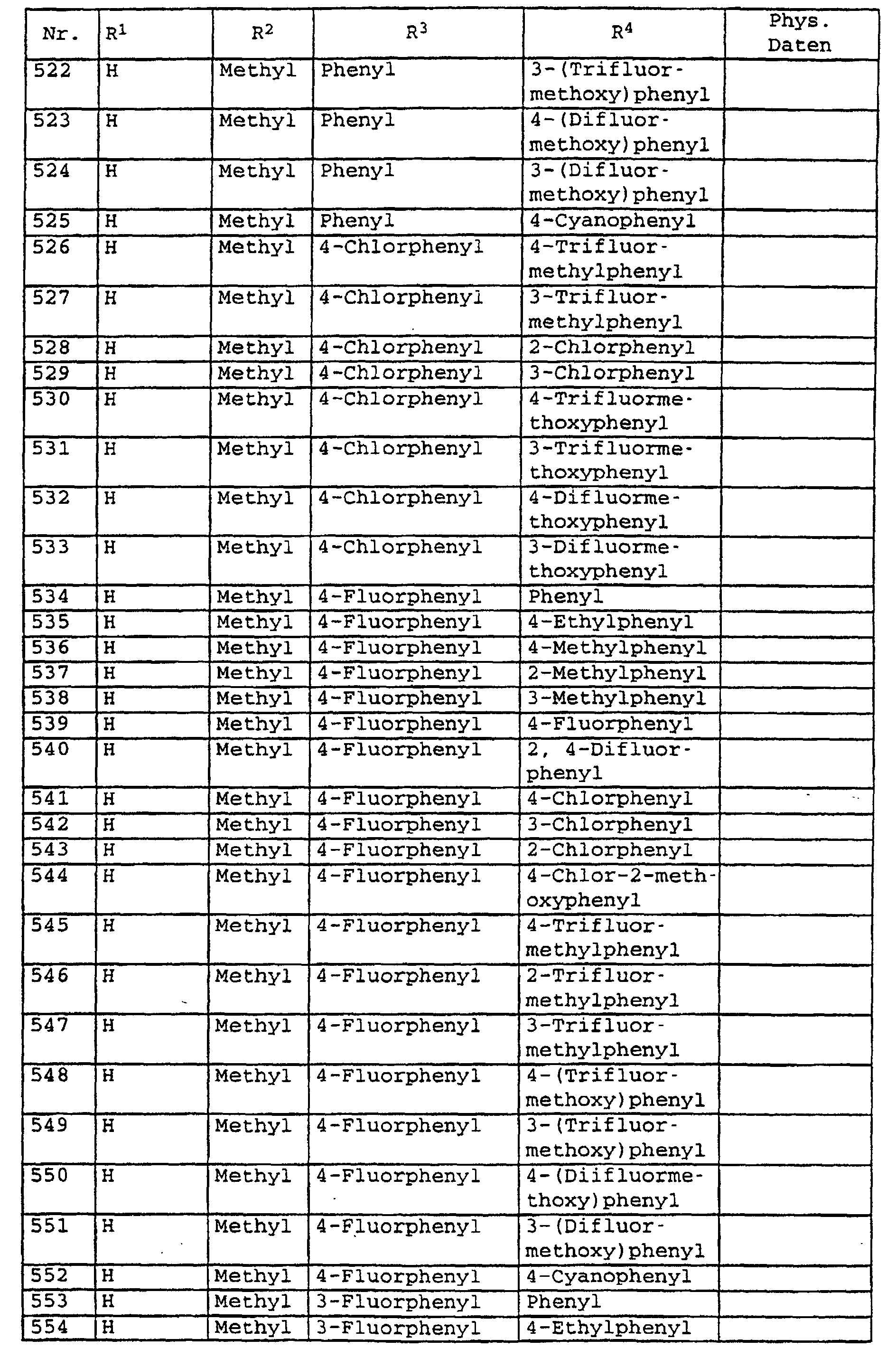

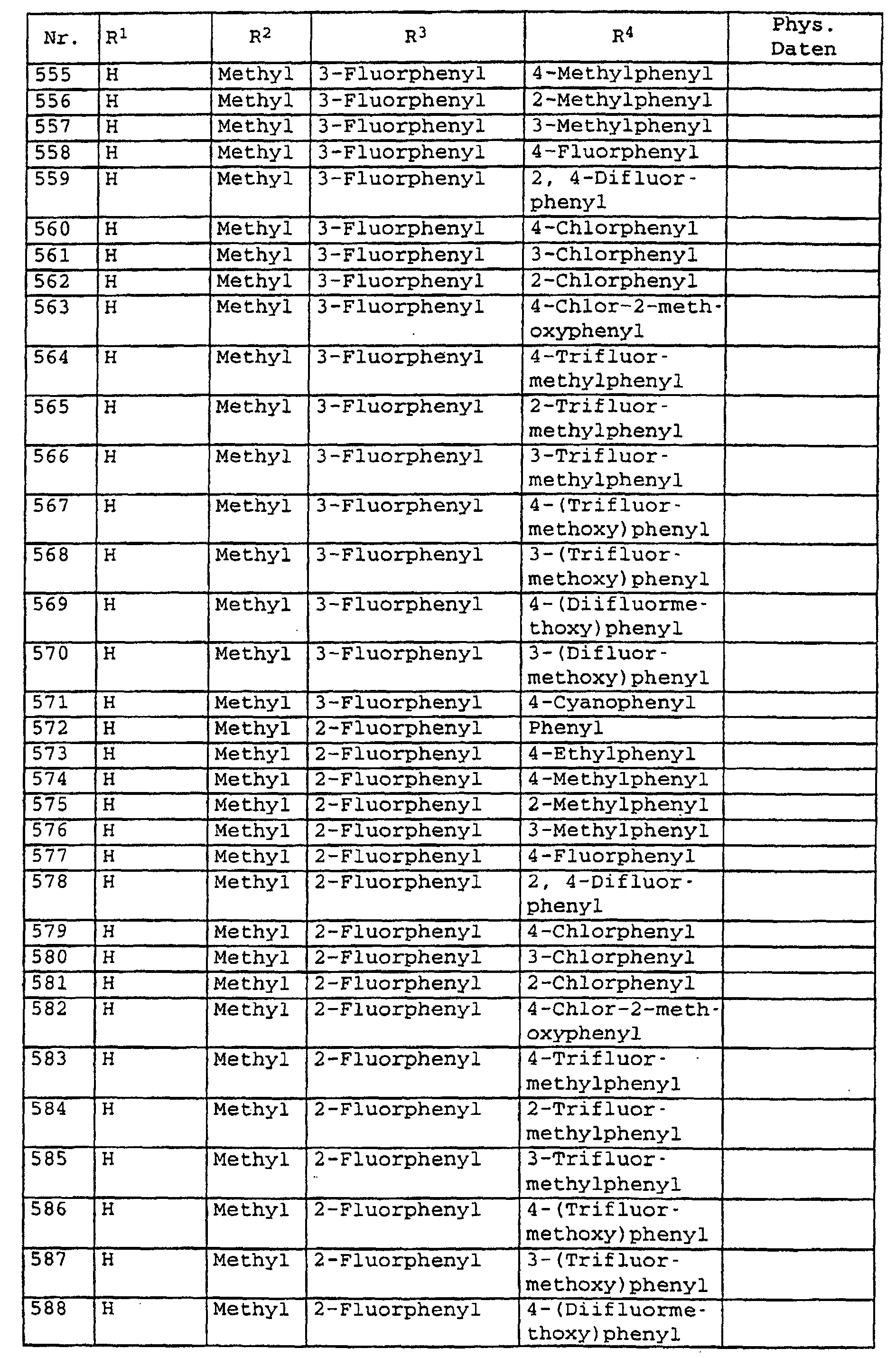

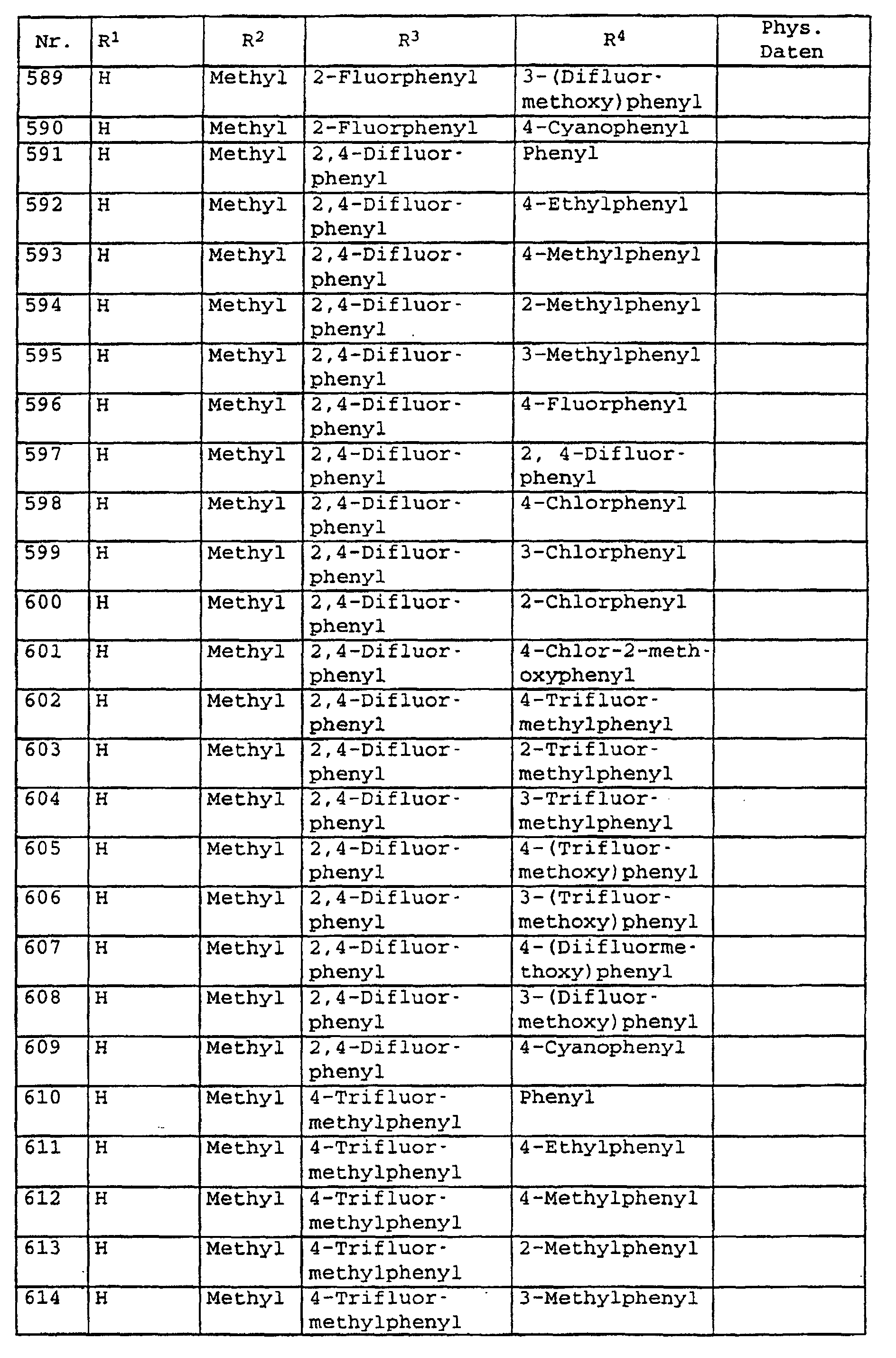

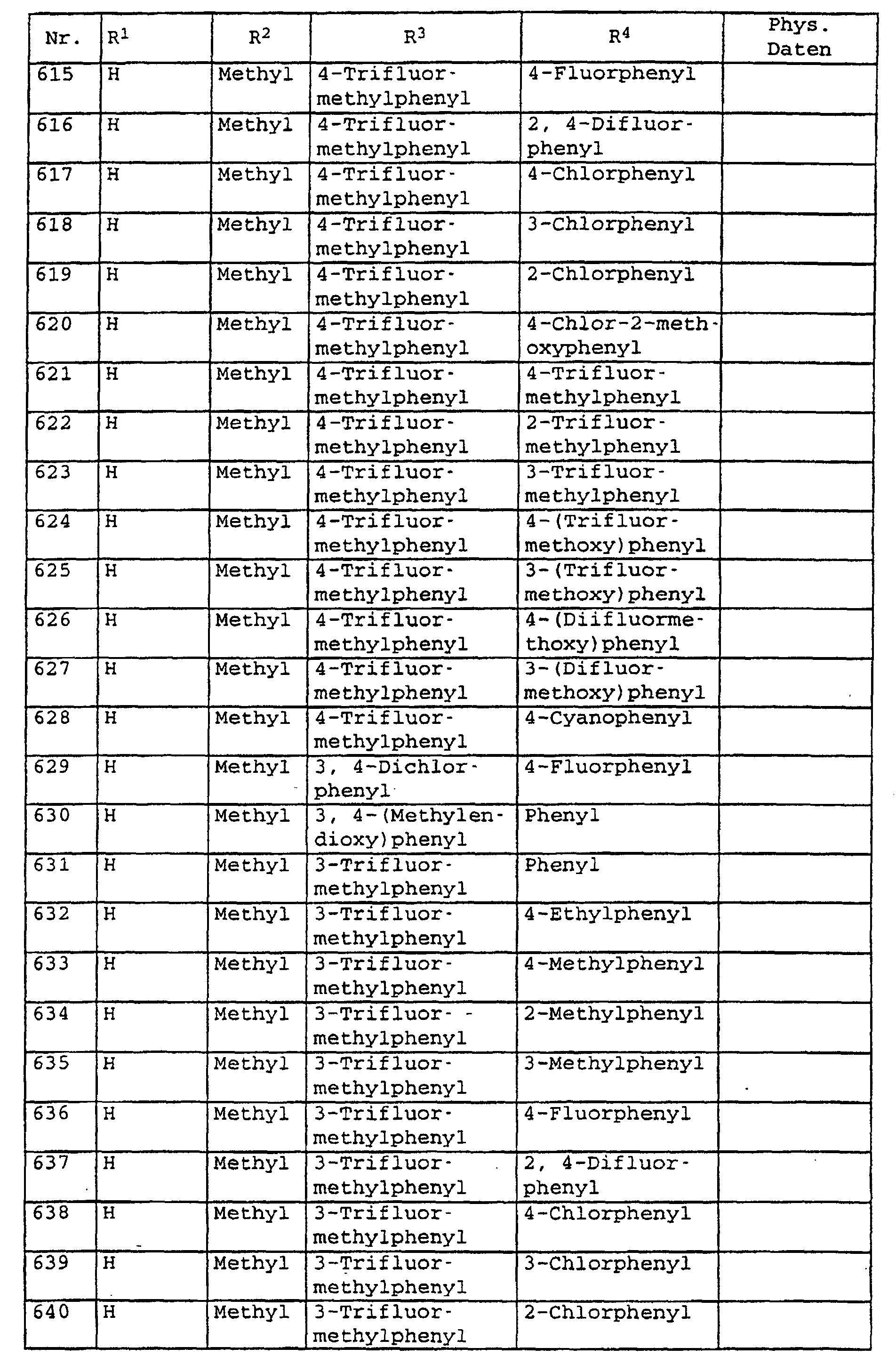

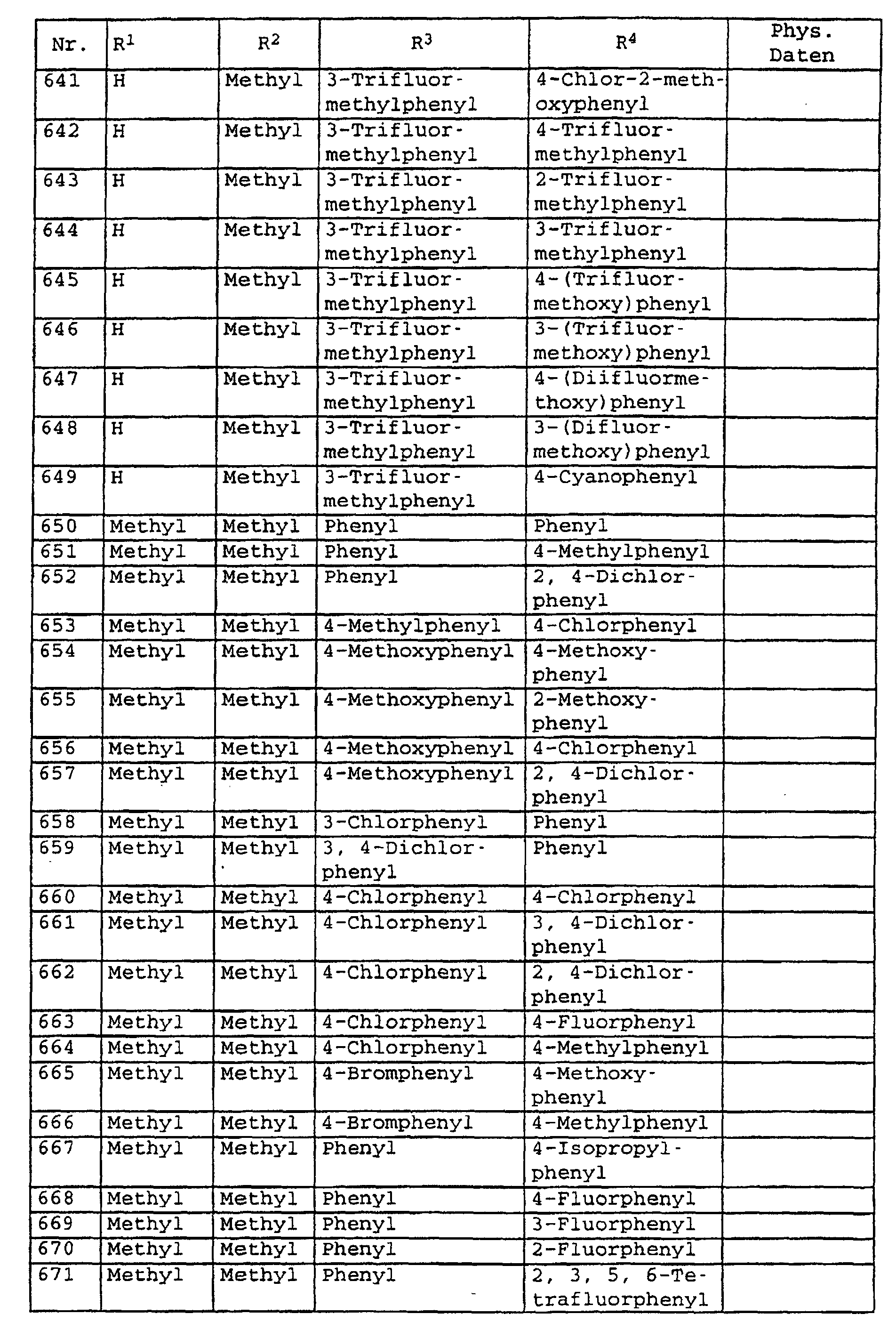

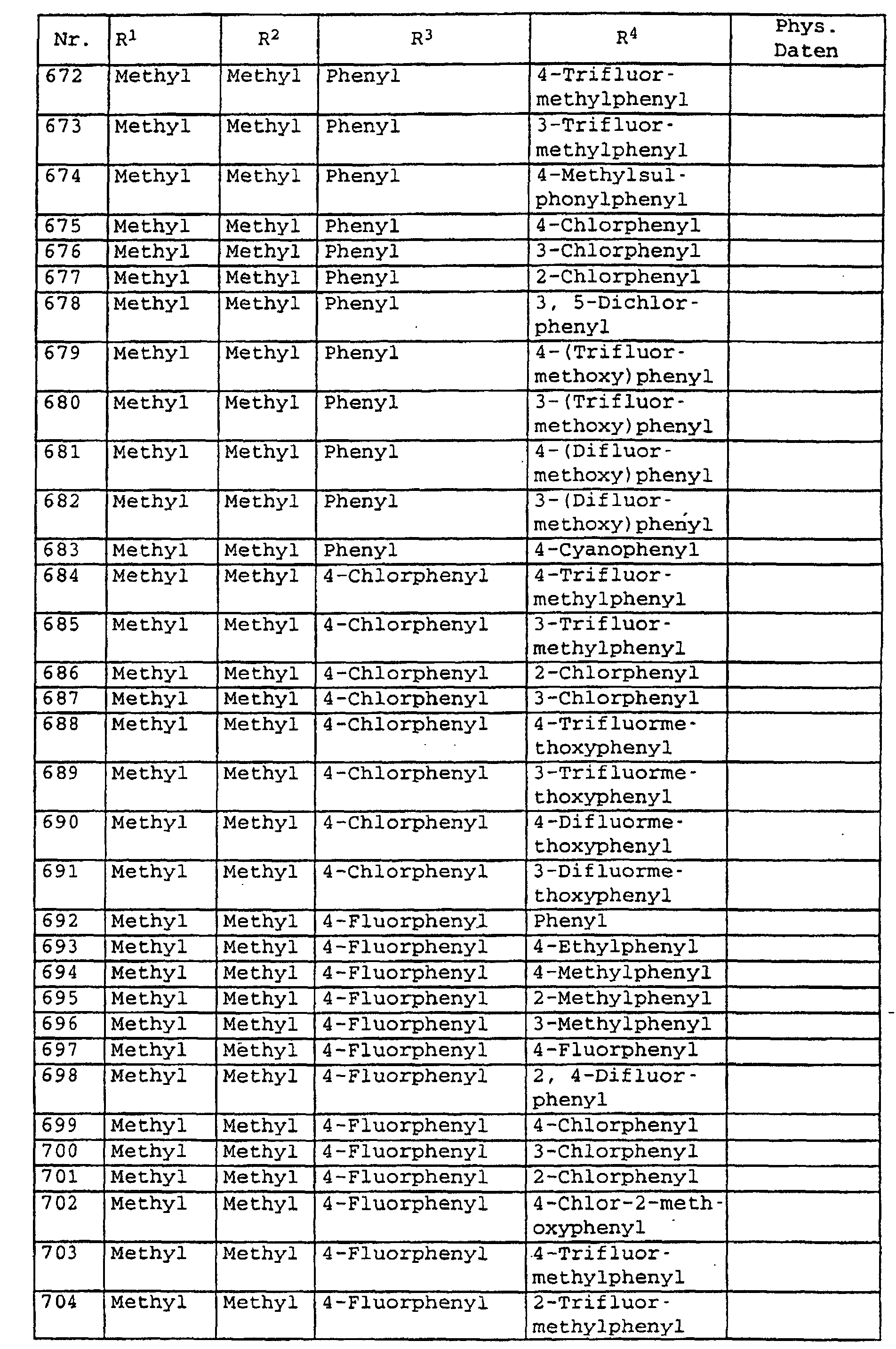

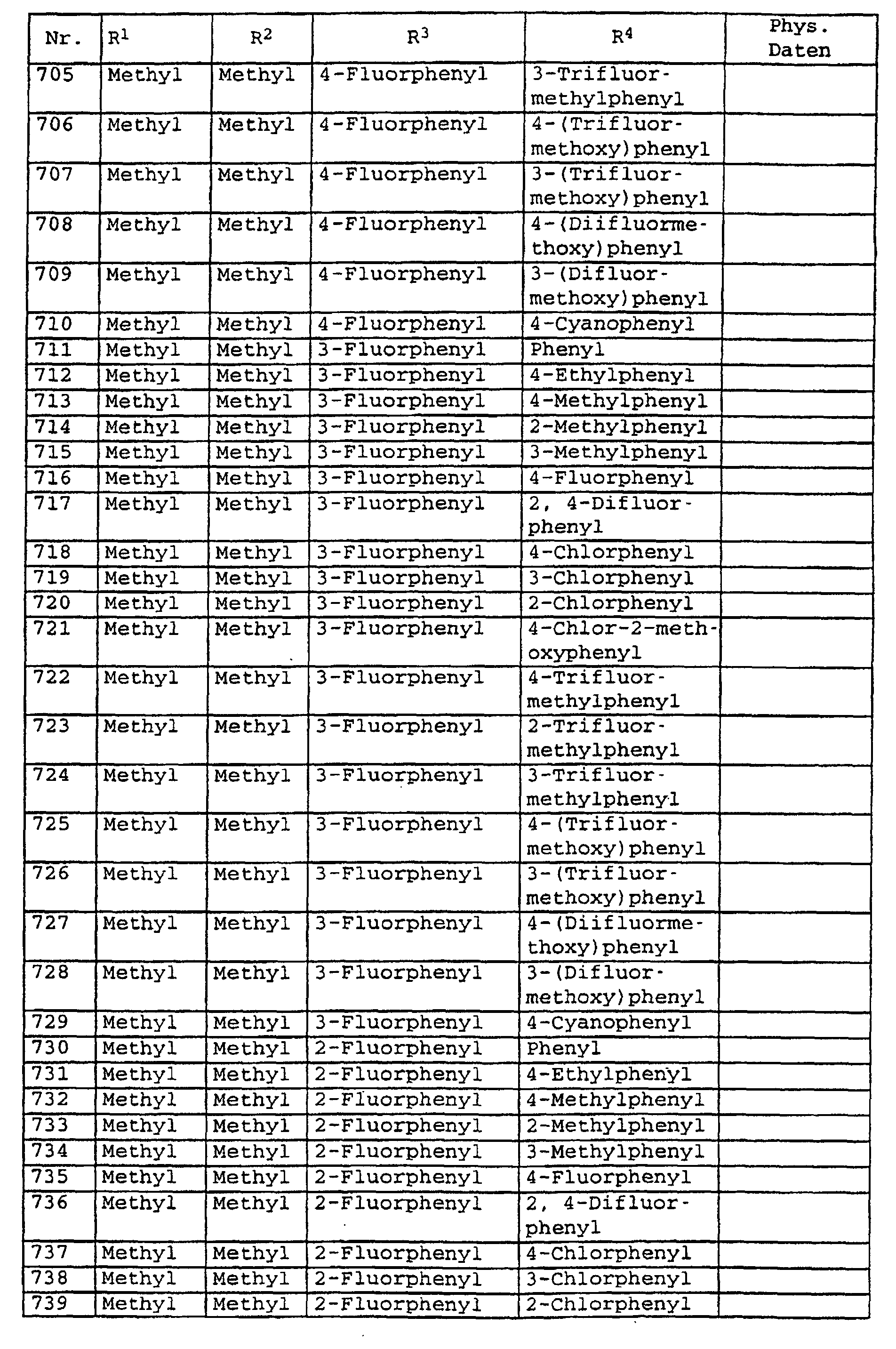

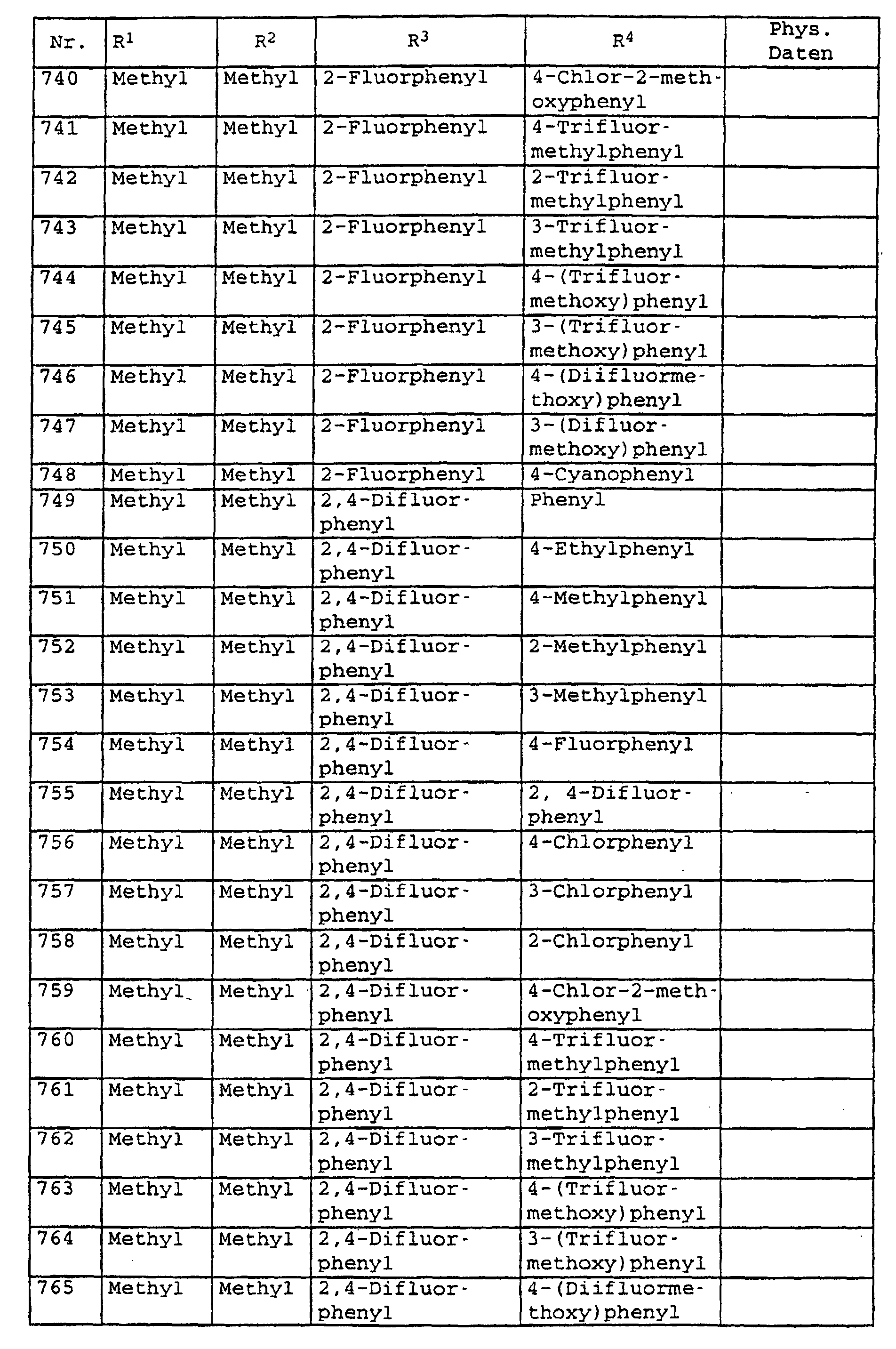

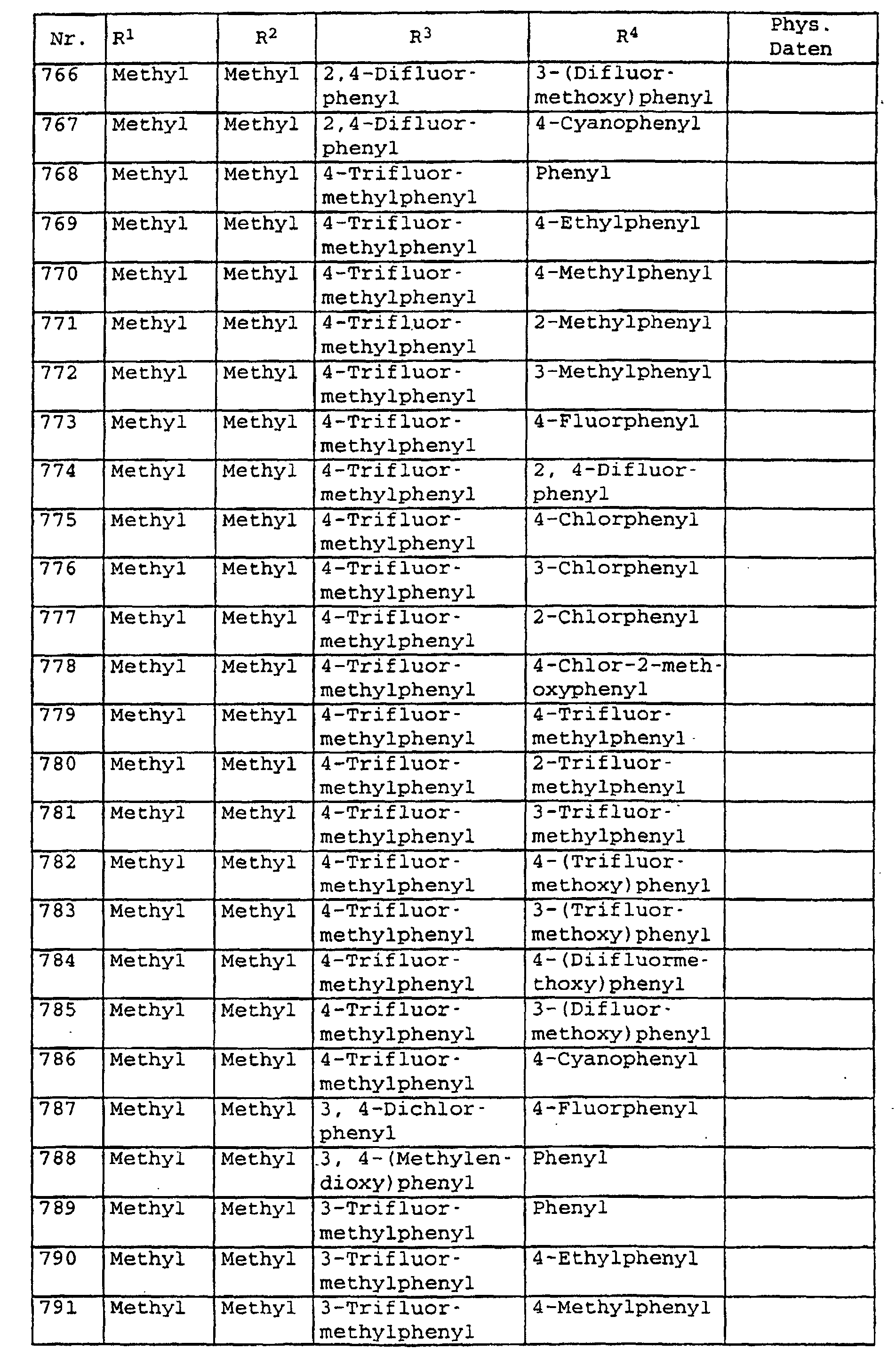

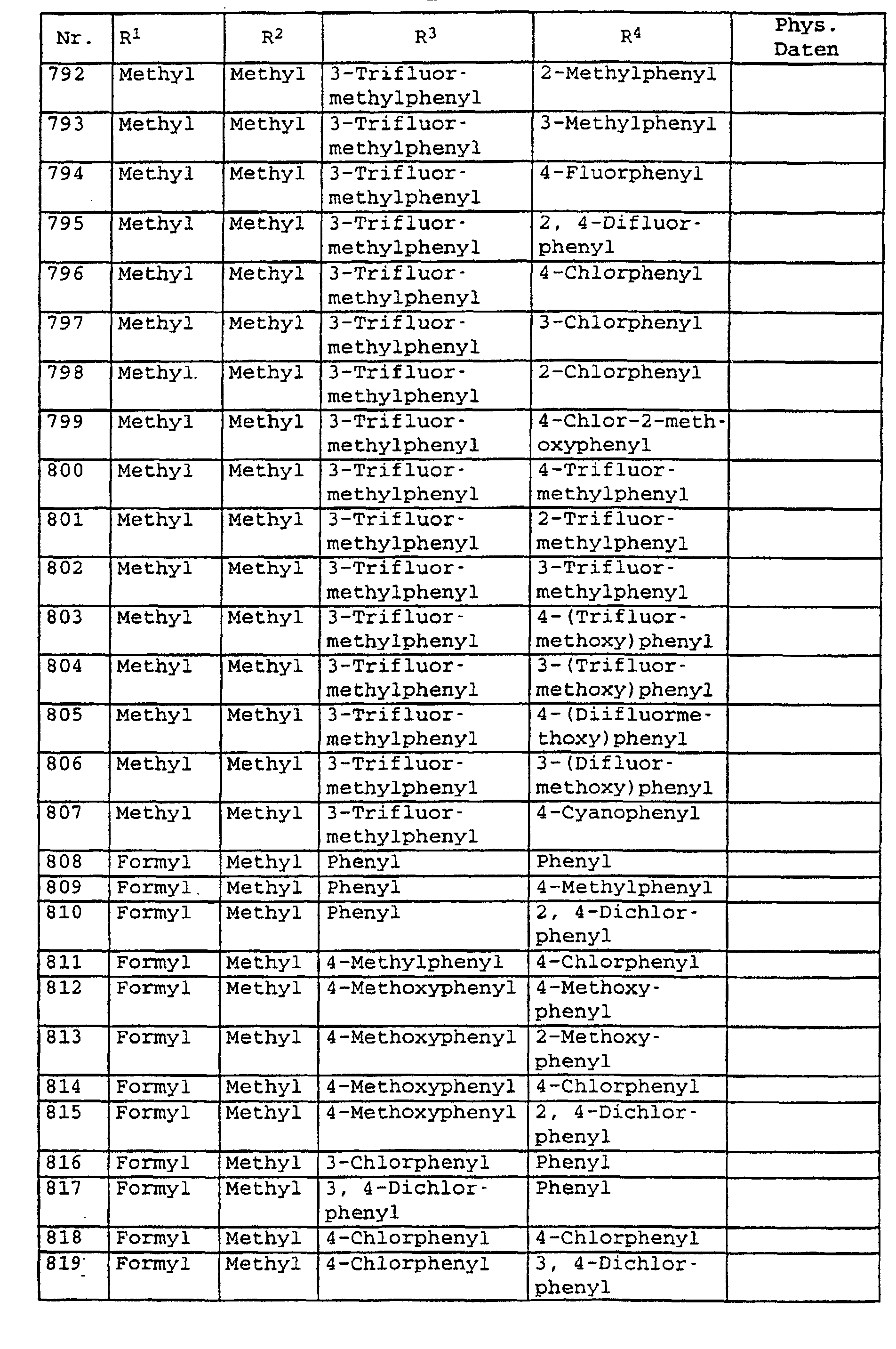

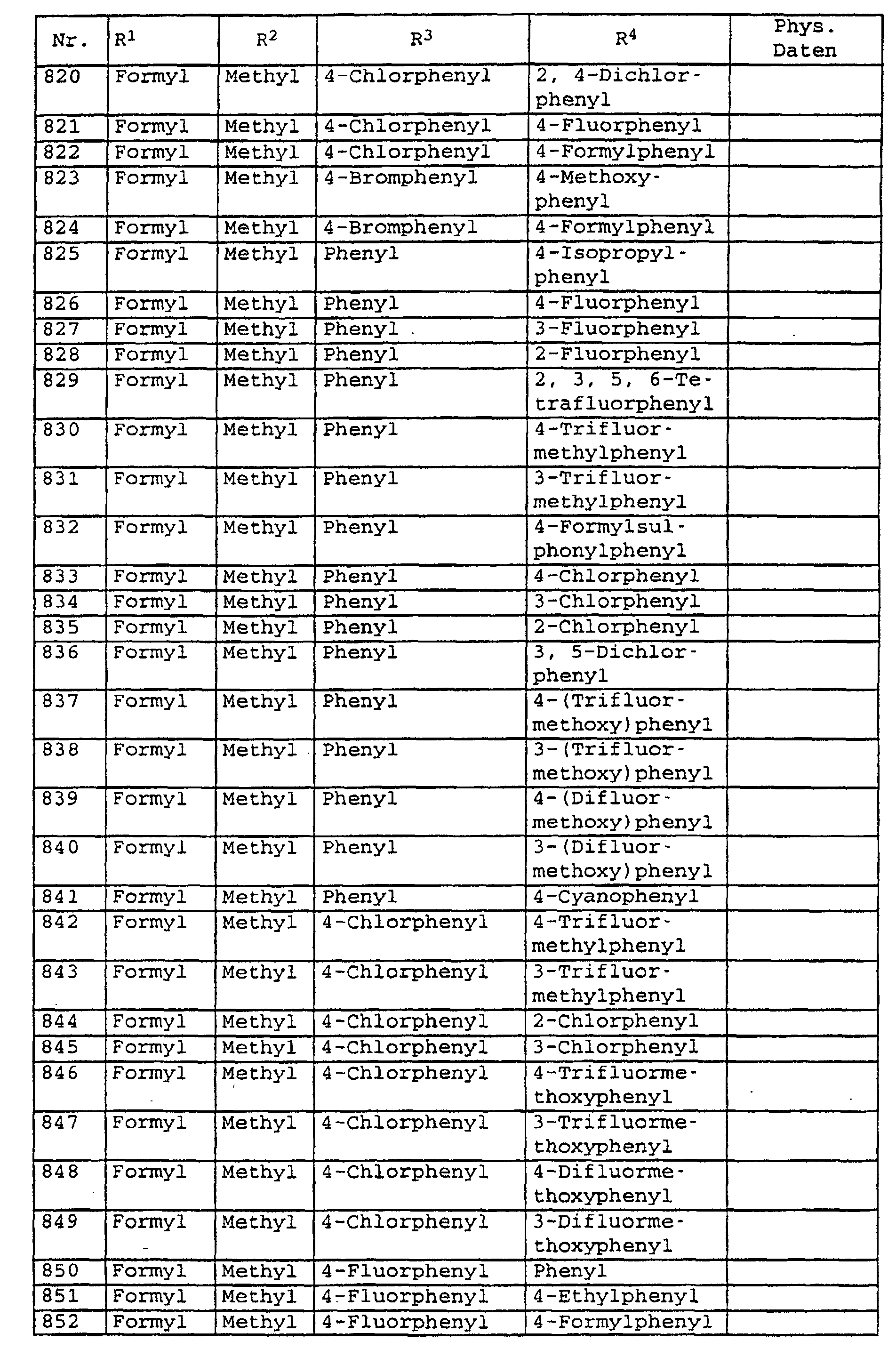

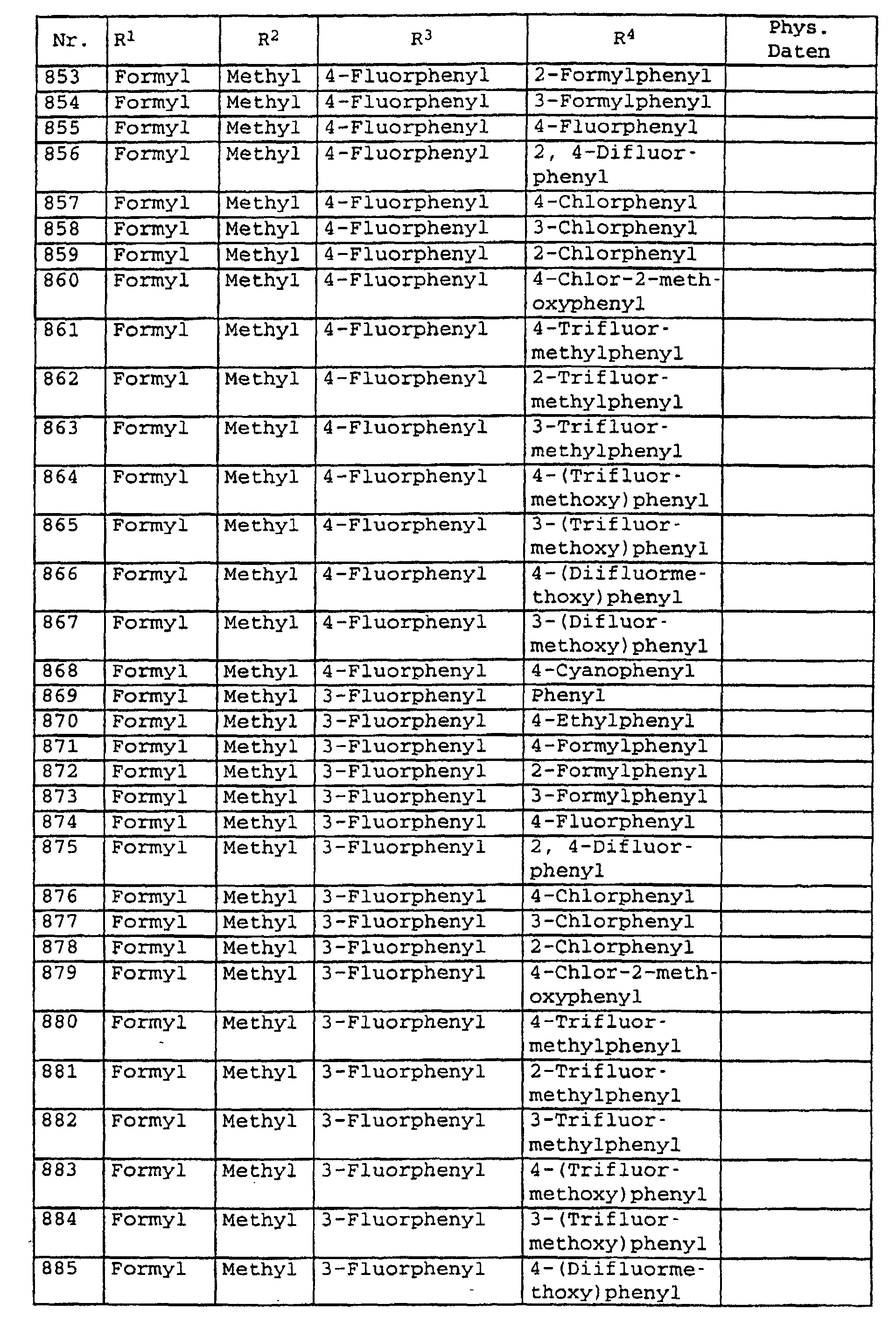

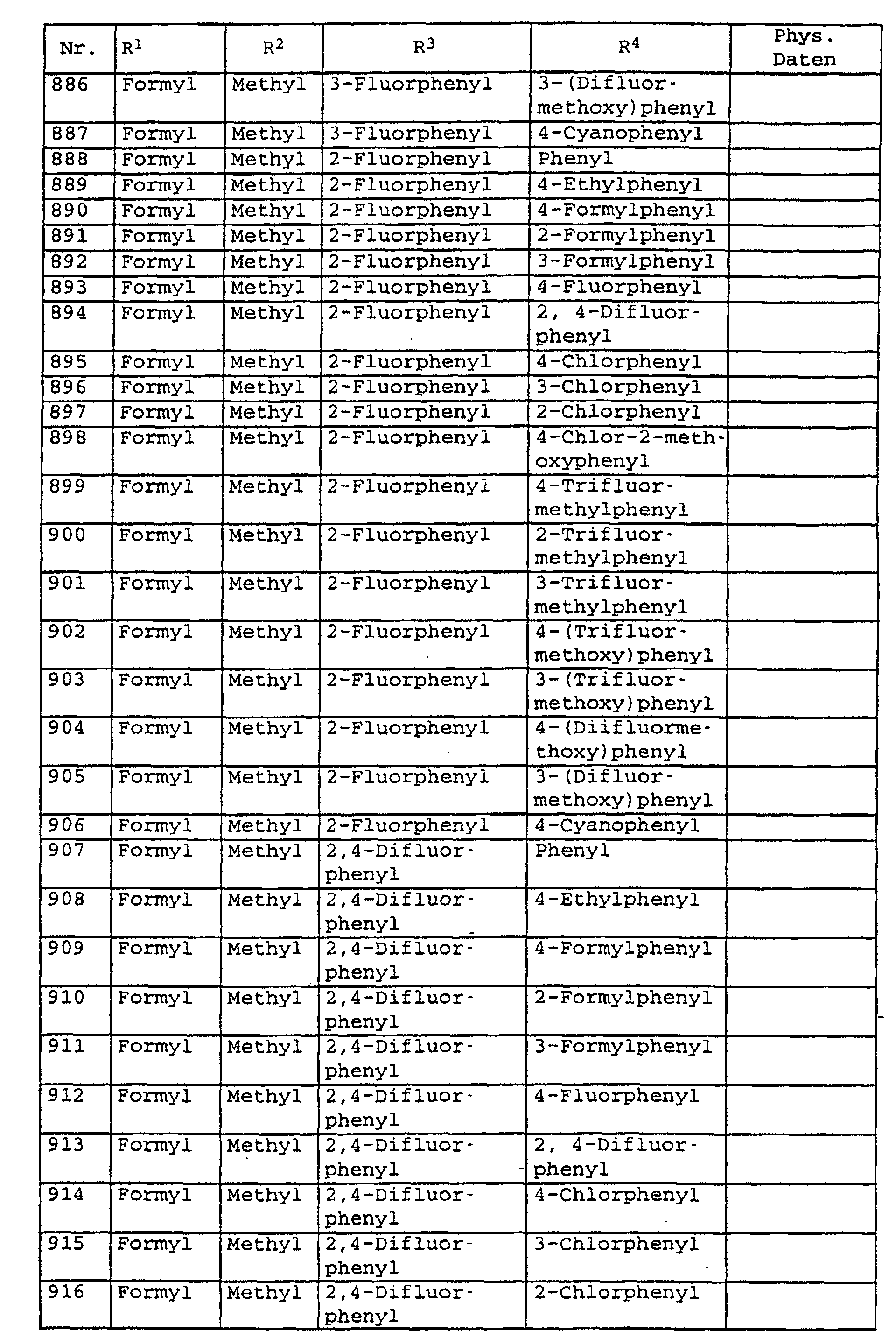

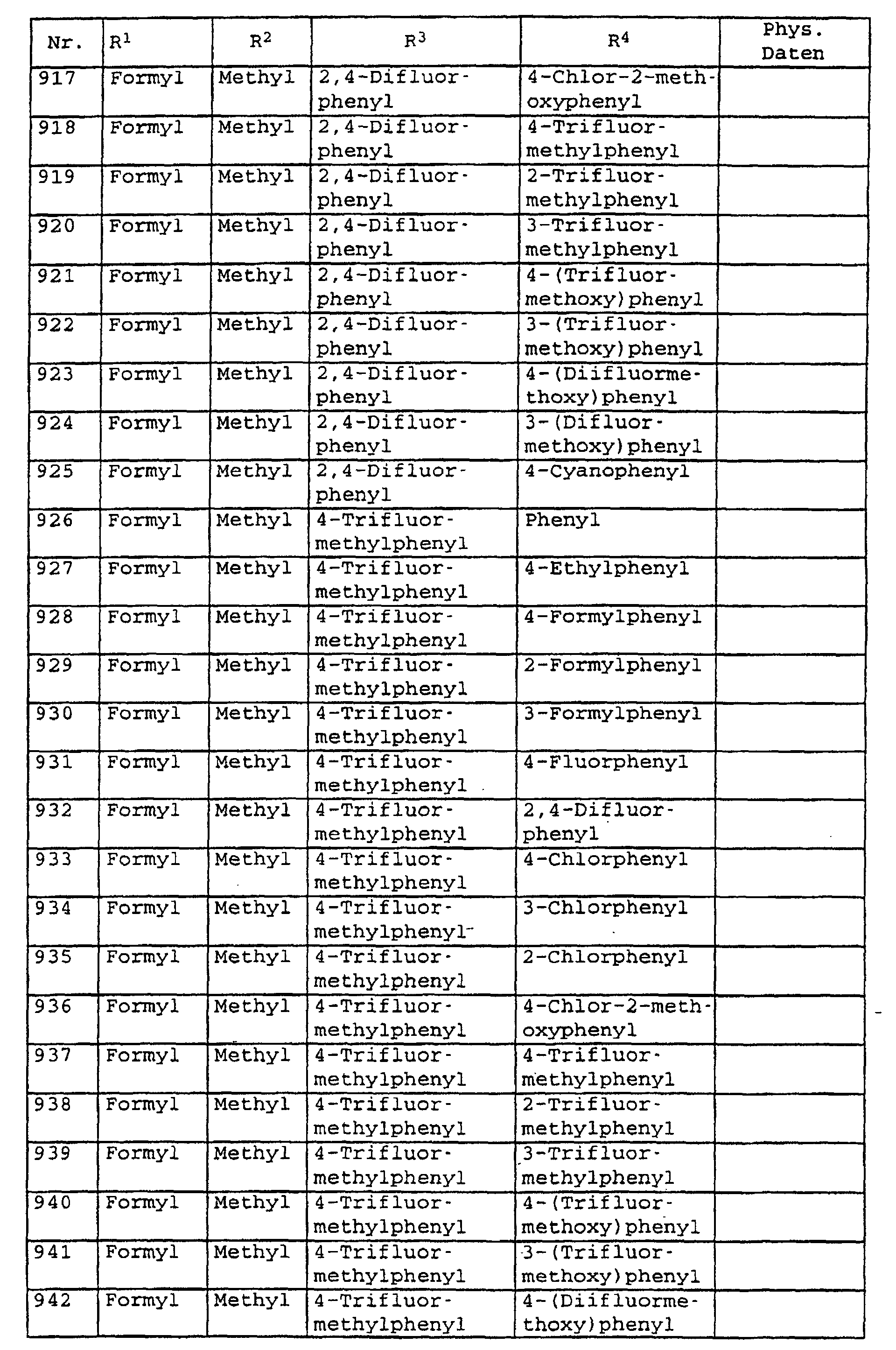

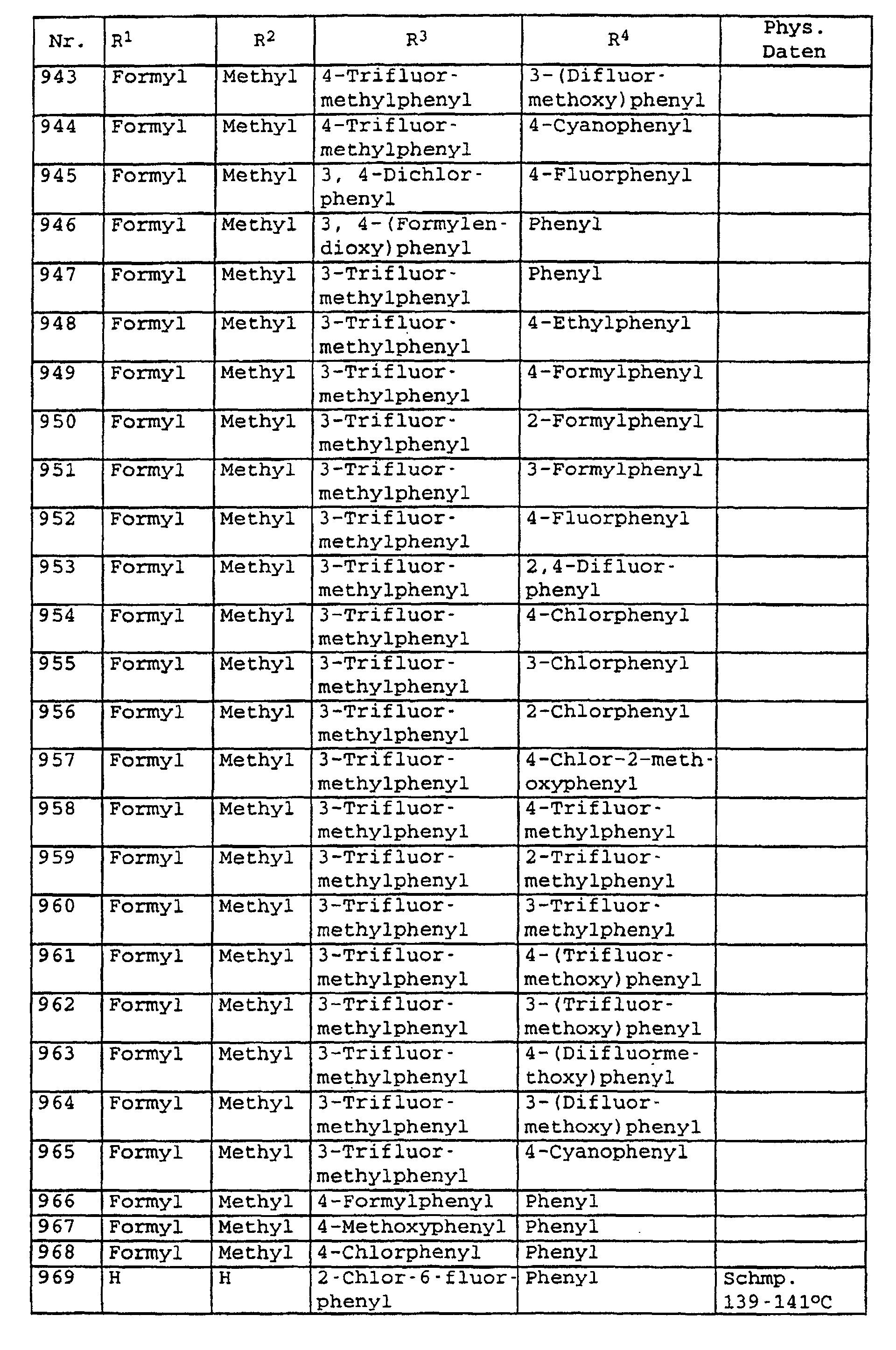

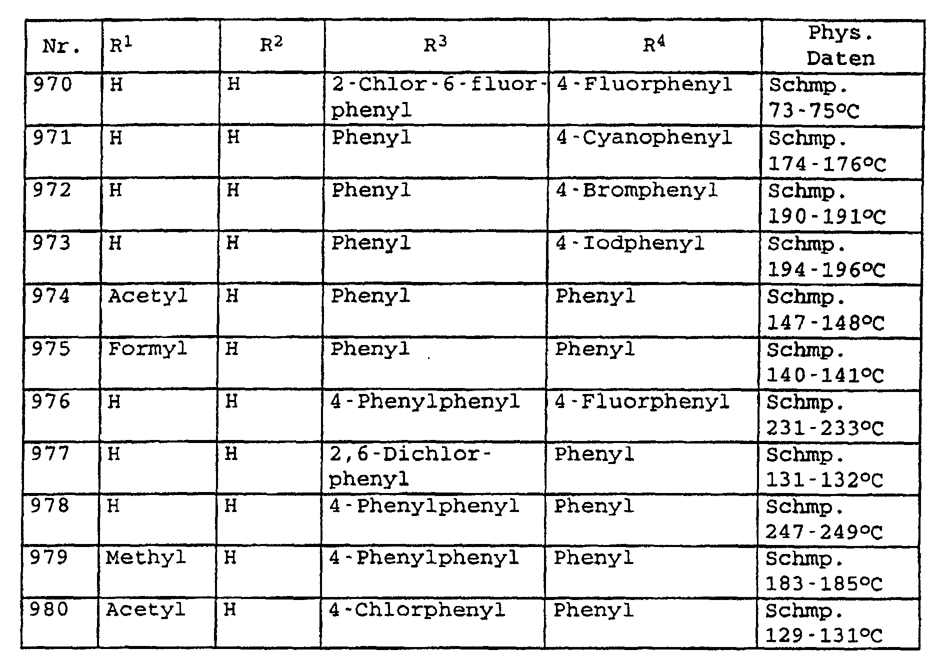

Die Verbindungen der Formel I lassen sich vorzugsweise anhand des folgenden Reaktionsschemas herstellen:

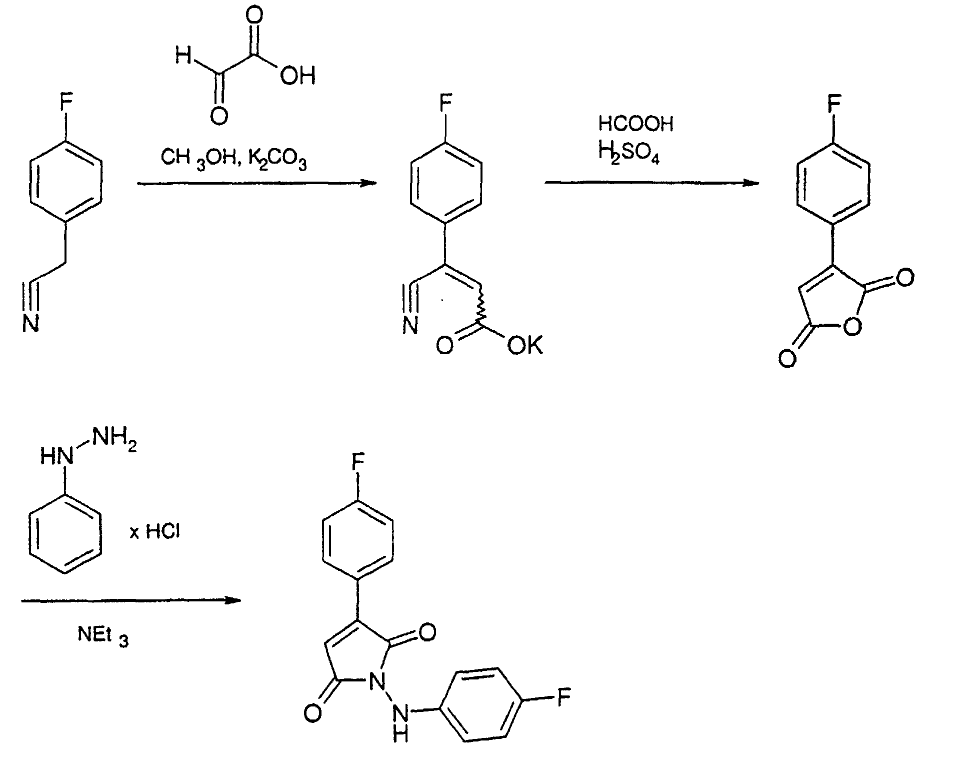

In Tabelle 2 sind verschiedene Verbindungen der Formel II dargestellt. Die in Tabelle 2 aufgelisteten Verbindungen der Formel II mit R₁= formyl stellen neue Verbindungen dar:

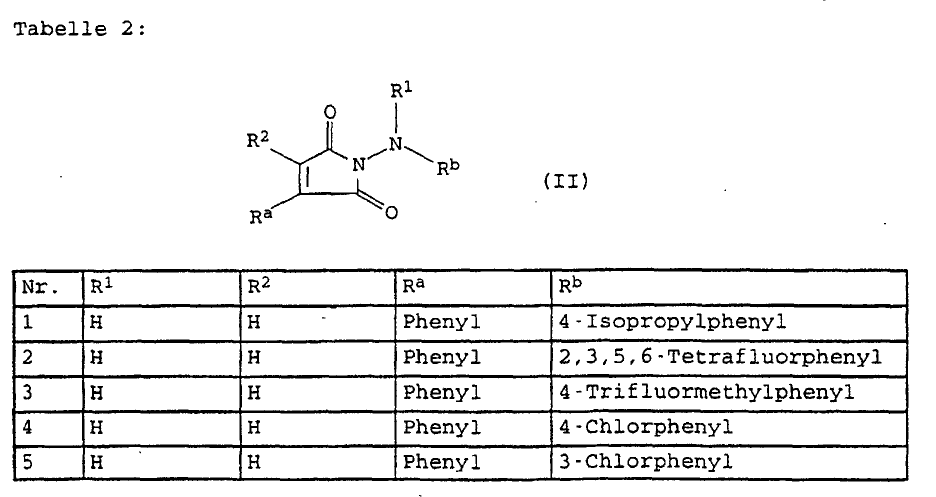

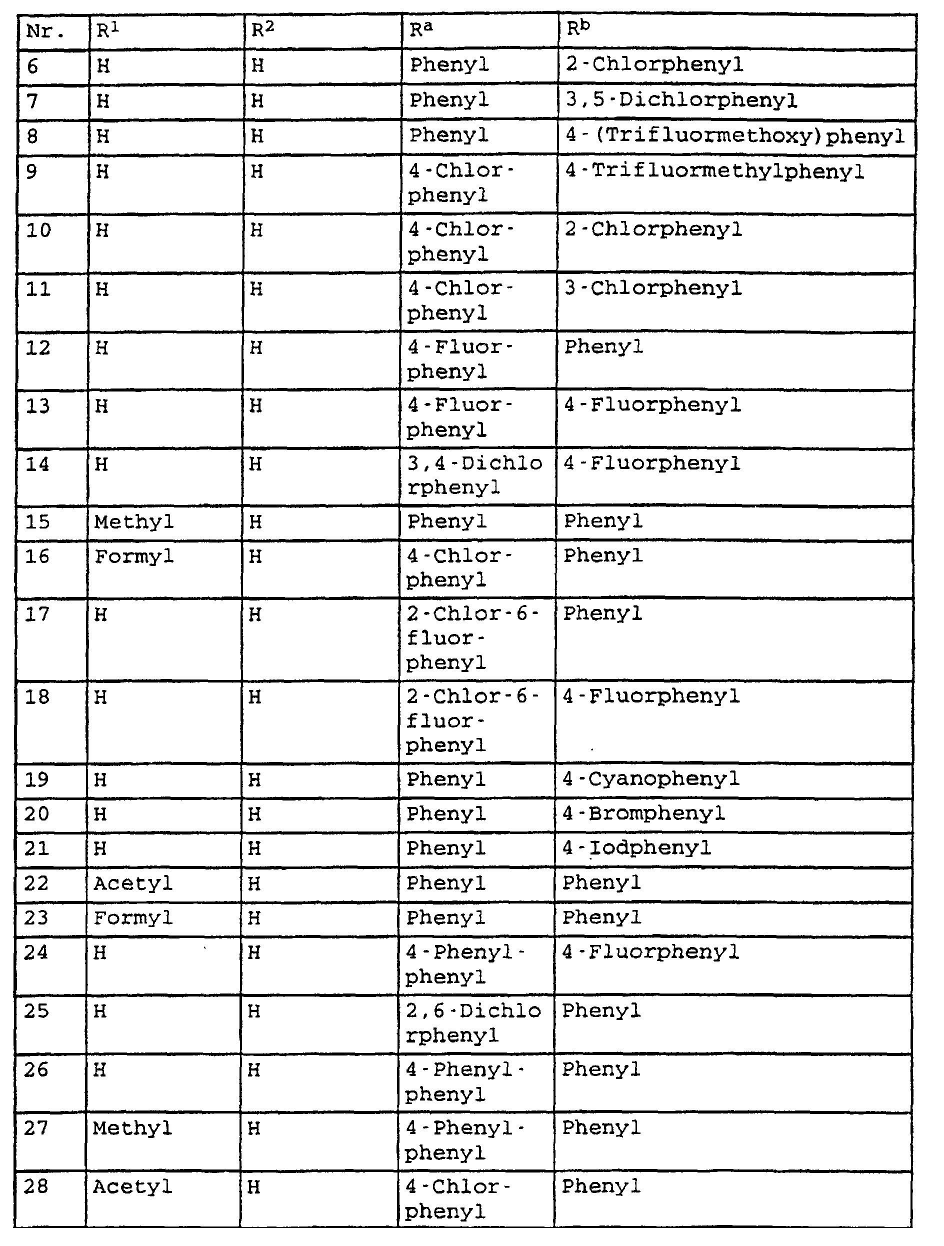

Im sinne der vorliegenden Erfindung Kommen insbesondere Verbindungen der Formel II bei denen R¹ eine Formylgruppe (-CHO) darstellt in Frage. Bevorzugt sind ferner Verbindungen II, bei denen die Reste R², R^{a} und R^{b} unabhängig voneinander folgende Bedeutung haben:
- R²: Wasserstoff;
- R^{a}: Phenyl, das ein- oder mehrfach, vorzugsweise ein- oder zweifach substituiert sein kann durch Halogen oder durch eine Phenylgruppe, die ihrerseits ebenfalls durch Halogen oder C₁-C₄-Alkyl substituiert sein kann;
- R^{b}: Phenyl, das ein- oder mehrfach, vorzugsweise ein- bis vierfach, substituiert sein kann durch Halogen, C₁-C₆-Alkyl, C₁-C₄-Halogenalkyl, C₁-C₄-Halogenalkoxy.

In diesem Sinne haben die Reste R¹, R^{a} und R^{b} im Fall der Verbindungen II beispielsweise folgende Bedeutung:
- R¹:: Formyl;
- R²:: Wasserstoff;
- R^{a}:: Phenyl, 4-Chlorphenyl, 4-Fluorphenyl, 3,4-Dichlorphenyl, 2-Chlor-6-fluorphenyl, 4-Phenylphenyl, 2,6-Dichlorphenyl oder 2-Chlorphenyl.
- R^{b}:: 4-isopropylphenyl, 2,3,5,6-Tetrafluorphenyl, 4-Trifluormethylphenyl, 4-Chlorphenyl, 3-Chlorphenyl, 2-Chlorphenyl, 3,5-Dichlorphenyl, 4-(Trifluormethoxy)phenyl, 4-Trifluormethylphenyl, Phenyl, 4-Fluorphenyl, 4-Cyanophenyl, 4-Bromphenyl, 4-Iodphenyl.

Die Verbindungen I zeichnen sich durch eine hervorragende fungizide Wirkung aus. Dies trifft insbesondere zu für die in der Tabelle 1 genannten Verbindungen Nr. 1, 13, 14, 17, 23, 26, 30, 31, 34, 35, 36, 38, 43, 45, 46, 51, 56, 969, 970, 971, 972, 973 und 977. Bevorzugte Verbindungen sind vor allem die folgenden: Nr. 13, 14, 23, 26, 31, 35, 36, 38, 46, 56, 969, 970, 971, 972 und 973.

Normalerweise werden die Pflanzen mit den Wirkstoffen besprüht oder bestäubt oder die Samen der Pflanzen mit den Wirkstoffen behandelt.

Die Formulierungen (fungiziden Mittel bzw. agrochemischen Zusammensetzungen) werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes iso-Octyl-, Octyl- oder Nonylphenol, Alkylphenol-, Tributylphenylpolyglykolether, Alkylarylpolyetheralkohole, iso-Tridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calciumund Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
II. eine Mischung aus 10 Gew.-Teilen einer erfindungsgemäßen Verbindung I, 70 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 10 Gew.-Teilen einer erfindungsgemäßen Verbindung I, 40 Gew.-Teilen Cyclohexanon, 30 Gew.-Teilen iso-Butanol, 20 Gew.-Teilen des Anlagerungsproduktes von 40 mol Ethylenoxid an 1 mol Ricinusöl;
IV. eine wäßrige Dispersion aus 10 Gew.-Teilen einer erfindungsgemäßen Verbindung I, 25 Gew.-Teilen Cyclohexanol, 55 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen, vorzugsweise einer festen erfindungsgemäßen Verbindung I, 3 Gew.-Teilen des Natriumsalzes der Di-iso-butylnaphthalin-2-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 62 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 50 Gew.-Teilen eines paraffinischen Mineralöls.

Die Wirkstoffe der Formel I zeichnen sich durch eine hervorragende Wirkung gegen ein breites Spektrum von pflanzenpathogenen Pilzen, insbesondere aus der Klasse der Ascomyceten, Deuteromyceten, Phycomyceten und Basidiomyceten, aus. Sie sind z.T. systemisch wirksam und können daher auch als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Wirkstoffe der Formel I können entweder in freier Form oder in Form ihrer landwirtschaftlich einsetzbaren bzw. umweltverträglichen Salze vorliegen. Derartige Salze sind beispielsweise Säureadditionssalze mit anorganischen oder organischen Säuren, z.B. Salzsäure, Schwefelsäure, Essigsäure, u.a. Säuren.

Die Wirkstoffe der Formel I können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,025 und 2, vorzugsweise 0,1 bis 1 kg Wirkstoff pro ha.

Bei der Saatgutbehandlung werden im allgemeinen Wirkstoffmengen von 0,001 bis 50, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit anderen fungiziden Wirkstoffen erhält man dabei in vielen Fällen eine Vergrößerung des fungiziden Wirkungsspektrums. Die Wirkstoffe der Formel I reduzieren insbesondere dann, wenn sie in Kombination mit anderen fungiziden Wirkstoffen eingesetzt werden, die Gefahr einer Resistenzentwicklung im Vergleich zur Anwendung der Einzelwirkstoffe.

Für den Fall, daß die Kulturpflanzen oder das Saatgut mit Kombinationspräparaten von Wirkstoffen der Formel I und anderen fungiziden Wirkstoffen behandelt werden, kann diese Anwendung gleichzeitig oder zeitlich nacheinander erfolgen. Bei gleichzeitiger Anwendung der Wirkstoffe der Formel I mit anderen Fungiziden erfolgt dies zweckmäßigerweise durch Herstellung einer agrochemischen Mischung der beiden Wirkstoffe, wobei diese Mischung zur Behandlung der Kulturpflanzen oder des Saatgutes in üblicher Weise angewandt werden. Bei zeitlich nacheinander erfolgendem Einsatz der Wirkstoffe erfolgt dies zweckmäßigerweise durch Anwendung der Einzelwirkstoffe entweder in kurzem zeitlichem Abstand oder im Abstand von mehreren Tagen oder Wochen.

Durch diese kombinierte Anwendung kann insgesamt die Häufigkeit der Behandlung der Pflanzen oder des Saatgutes mit Fungiziden reduziert werden.

Im Sinne der vorliegenden Erfindung versteht man unter dem Begriff "Kombinationspräparate" grundsätzlich alle agrochemischen Zusammensetzungen, die Wirkstoffe der Formel I oder II sowie einen oder mehrere Wirkstoffe, insbesondere mit fungizider Wirkung enthalten, beispielsweise in Form von üblichen agrochemischen Mischungen. Der Begriff "Kombinationspräparate" umfaßt ferner auch solche agrochemischen Zubereitungen, die Wirkstoffe der Formel I enthalten, und die außerdem einen Hinweis enthalten, daß sich diese Wirkstoffe zur kombinierten Anwendung mit anderen Wirkstoffen auf dem Agrarsektor eignen. Ein derartiger Hinweis kann z.B. in Form eines Verpackungsaufdruckes auf der Handelsware oder auf dem Behältnis vorliegen, in dem sich der Wirkstoff der Formel I bzw. das agrochemische Mittel enthaltend einen Wirkstoff der Formel I befindet. Alternativ ist es auch möglich, daß andere agrochemische Erzeugnisse entsprechende Hinweise auf die kombinierte Anwendung mit Verbindungen der Formel I oder II aufweisen. Derartige Erzeugnisse gelten in diesem Sinne ebenfalls als Kombinationspräparate, die zur Anwendung in Kombination mit Wirkstoffen der Formel I bzw. II geeignet sind.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thion-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäureanilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan,2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-ylethyl]-1H-1,2,4-triazol, N-(n-Propyl)-N-(2,4,6-trichlorphenoxyethyl)-N'-imidazol-yl-harnstoff, 1- (4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol, sowie
verschiedene Fungizide, wie Dodecylguanidinacetat, 3-[3-(3,5-Dimethyl-2-oxycyclohexyl)-2-hydroxyethyl]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[(3,5-Dichlorphenyl)-5-methyl-5-methoxymethyl-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol,
Strobilurine wie Methyl-E-methoximino-[α-(o-tolyloxy)-o-tolyl]acetat, Methyl-E-2-{2-[6-(2-cyanophenoxy)pyridimin-4-yloxy]phenyl}-3-methoxyacrylat, Methyl-E-methoximino-[α-(2,5-dimethyloxy)-o-tolyl]acetamid,
Anilino-Pyrimidine wie N-(4,6-dimethylpyrimidin-2-yl)anilin, N-[4-methyl-6-(1-propinyl)pyrimidin-2-yl]anilin, N-(4-methyl-6-cyclopropyl-pyrimidin-2-yl)anilin,
Phenylpyrrole wie 4-(2,2-difluor-1,3-benzodioxol-4-yl)-pyrrol-3-carbonitril,
Zimtsäureamide wie 3-(4-chlorphenyl)-3-(3,4-dimethoxyphenyl)acrylsäuremorpholid.

Die Erfindung wird anhand der folgenden Ausführungsbeispiele näher erläutert:

### Beispiel 1

### 1-Anilino-3-(3,4-dichlorphenyl)-pyrrol-2,5-dion (Tabelle 1, Nr. 12)

### a) Kalium-(3-cyano-3-(3,4-dichlorphenyl)-acrylat)

18,6 g (0,1 Mol) (3,4-Dichlorphenyl)acetonitril und 35 g (0,25 Mol) Kaliumcarbonat wurden in 200 ml Methanol vorgelegt, mit 22,2 g (0,15 Mol) 50%iger wässriger Glyoxylsäure versetzt und 5 h bei Raumtemperatur gerührt. Der Rückstand wurde abfiltriert, mit Methylenchlorid gewaschen, mit 1 l Wasser über Nacht bei Raumtemperatur gerührt, abfiltriert und das ausgefallene Produkt mit Wasser gewaschen und getrocknet. Ausbeute 26,2 g, Schmp. 235-238°C.

### b) (3, 4-Dichlorphenyl)-furan-2,5-dion

25,8 g (92 mmol) Kalium-(3-cyano-3-(3,4-dichlorphenyl)-acrylat) wurden in 200 ml 88 %iger Ameisensäure gelöst und tropfenweise mit 15 ml konzentrierter Schwelsäure versetzt. Dann wurde 3 h unter Rückfluß erhitzt, bei 90°C in 2,5 l Wasser gegossen, 1 h nachgerührt, das Produkt abfiltriert, mit Wasser gewaschen und getrocknet. Ausbeute: 18,9 g, Schmp. 112°C.

### c) 1-Anilino-3-(3,4-dichlorphenyl)-pyrrol-2,5-dion

2,43 g (10 mmol) -(3, 4-Dichlorphenyl)-furan-2,5-dion und 1,08 g (10 mmol) Phenylhydrazin wurden in 50 ml Eisessig 5 h unter Rückfluß gekocht. Nach dem Abkühlen wurde das Produkt abfiltriert und zuerst mit Eisessig und anschließend mit Pentan gewaschen. Ausbeute: 1,8 g eines kristallinen Feststoffs, Schmp. 208-210°C.

### Beispiel 2

### Wirksamkeit gegen Phytophthora infestans an Tomaten

Blätter von Topfpflanzen der Sorte "Große Fleischtomate" wurden mit einer wäßrigen Suspension, die aus einer Stammlösung aus 10 % Wirkstoff, 63 % Cyclohexanon und 27 % Emulgiermittel angesetzt wurde, bis zur Tropfnässe besprüht. Am folgenden Tag wurden die Blätter mit einer wäßrigen Zoosporenaufschwemmung von *Phytophthora infestans* infiziert. Anschließend wurden die Pflanzen in einer wasserdampfgesättigten Kammer bei Temperaturen zwischen 16 und 18°C aufgestellt. Nach 6 Tagen hatte sich die Krautfäule auf den unbehandelten, jedoch infizierten Kontrollpflanzen so stark entwickelt, daß der Befall visuell in % ermittelt werden konnte.

| Wirkstoff | %-Befall der Blätter nach Applikation von 250 ppm-haltiger wäßriger Wirkstoffaufbereitung |
|---|---|
| Tabelle 1. Nr. 1 | 15 |
| Tabelle 1. Nr. 13 | 2 |
| Tabelle 1. Nr. 17 | 5 |
| Tabelle 1. Nr. 31 | 7 |
| Tabelle 1. Nr. 34 | 5 |
| Tabelle 1. Nr. 485 | 10 |
| Tabelle 1. Nr. 969 | 3 |
| Tabelle 1. Nr. 971 | 3 |
| Tabelle 1. Nr. 972 | 5 |
| Tabelle 1. Nr. 973 | 8 |
| Unbehandelt | 90 |

Aus der vorstehenden Tabelle geht hervor, daß bei den behandelten Pflanzen eine durch Schadpilze verursachte deutlich geringere Schädigung zu beobachten ist als im Vergleich zu den unbehandelten Pflanzen. Die erfindungsgemäßen Wirkstoffe weisen demzufolge eine gute fungizide Wirkung auf. Sie besitzen insbesondere einen protektiven Effekt gegen Schadpilze.

### Beispiel 3

### Wirksamkeit gegen Plasmopara viticola

Blätter von Topfreben der Sorte "Müller-Thurgau" wurden mit wäßriger Wirkstoffaufbereitung, die mit einer Stammlösung aus 10 % Wirkstoff, 63 % Cyclohexanon und 27 % Emulgiermittel angesetzt wurde, bis zur Tropfnässe besprüht. Um die Dauerwirkung der Substanzen beurteilen zu können, wurden die Pflanzen nach dem Antrocknen des Spritzbelages für 7 Tage im Gewächshaus aufgestellt. Erst dann wurden die Blätter mit einer wäßrigen Zoosporenauf schwemmung von *Plasmopara viticola* inokuliert. Danach wurden die Reben zunächst für 48 Stunden in einer wasserdampfgesättigten Kammer bei 24° C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

| Wirkstoff | %-Befall der Blätter nach Applikation von 250 ppm-haltiger wäßriger Wirkstoffaufbereitung |
|---|---|
| Tabelle 1. Nr. 1 | 5 |
| Tabelle 1. Nr. 17 | 5 |
| Tabelle 1. Nr. 30 | - 15 |
| Tabelle 1. Nr. 34 | 5 |
| Tabelle 1. Nr. 43 | 15 |
| Tabelle 1. Nr. 45 | 10 |
| Tabelle 1. Nr. 51 | 15 |
| Tabelle 1. Nr. 969 | 5 |
| Tabelle 1. Nr. 971 | 10 |
| Tabelle 1. Nr. 972 | 1 |
| Tabelle 1. Nr. 973 | 1 |
| Tabelle 1. Nr. 977 | 3 |
| Unbehandelt | 85 |

## Patentansprüche

1. Agrochemische Zusammensetzung mit fungizider Wirkung enthaltend als Wirkstoffe Verbindungen der Formel I 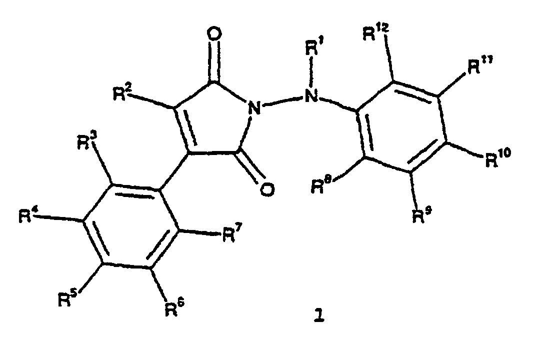 wobei die Reste folgende Bedeutungen haben:
R¹ Wasserstoff, C₁-C₆-Alkyl, C₁-C₆-Alkylcarbonyl, Formyl oder C₁-C₆-Halogenalkylcarbonyl,
R² Wasserstoff, C₁-C₆-Alkyl oder C₁-C₆-Halogenalkyl;
R³ - R¹² Wasserstoff, Halogen, C₁-C₈-Cycloalkyl, C₁-C₆-Alkyl, C₁-C₆-Halogenalkyl, C₁-C₆-Alkoxy, C₁-C₆-Halogenalkoxy, C₁-C₆-Alkylsulfonyl, C₁-C₆-Halogenalkylsulfonyl, Formyl, C₁-C₆-Alkylcarbonyl, Cyano, C₁-C₆-Alkylthio oder Phenyl, welches ggf. durch Halogenatome, C₁-C₆-Alkyl- oder C₁-C₆-Halogenalkyl-Gruppen substituiert sein kann, sowie deren landwirtschaftlich einsetzbaren Salze.

2. Zusammensetzung nach Anspruch 1, wobei R¹ Wasserstoff, C₁-C₆-Alkyl oder Formyl bedeutet.

3. Zusammensetzung nach Anspruch 2, wobei R¹ Wasserstoff oder Methyl bedeutet.

4. Zusammenfassung nach einem der Ansprüche 1 - 3, wobei R² Wasserstoff, C₁-C₆-Alkyl oder Trifluormethyl bedeutet.

5. Zusammensetzung nach Anspruch 4, wobei R² Wasserstoff bedeutet.

6. Zusammensetzung nach einem der Ansprüche 1 - 5, wobei ein oder mehrere der Reste R³-R¹² die folgenden Bedeutungen haben: Fluor, Chlor, Methyl, Ethyl, Propyl, Butyl, Trifluormethyl, Difluormethyl, Trifluormethoxy, Difluormethoxy, Methoxy, Methylthio, Cyano, und die übrigen Reste R³-R¹² Wasserstoff bedeuten.

7. Zusammensetzung nach Anspruch 6, wobei vier bis neun der Reste R³-R¹² Wasserstoff bedeuten.

8. Zusammensetzung nach einem der Ansprüche 1-7, wobei zwei bis fünf der Reste R⁸-R¹² Wasserstoff bedeuten.

9. Zusammensetzung nach Anspruch 8, wobei drei oder vier der Reste R⁸-R¹² Wasserstoff bedeuten.

10. Zusammensetzung nach einem der Ansprüche 1-9, wobei zwei bis fünf der Reste R³-R⁷ Wasserstoff bedeuten.

11. Zusammensetzung nach Anspruch 10, wobei drei oder vier der Reste R³-R⁷ Wasserstoff bedeuten.

12. Zusammensetzung nach einem der Ansprüche 1 - 11, wobei mindestens zwei der Reste R⁸-R¹² sowie mindestens zwei der Reste R³-R⁷ Wasserstoff bedeuten.

13. Zusammensetzung nach einem der Ansprüche 1 - 12, wobei einer, zwei oder drei der Reste R³-R¹² Halogen, C₁-C₆-Alkyl, C₁-C₆-Halogenalkyl, C₁-C₆-Alkoxy oder C₁-C₆-Halogenalkoxy bedeuten.

14. Zusammensetzung nach Anspruch 13, wobei die Reste R³-R¹² ausgewählt sind aus Fluor, Chlor, Brom, Jod, Methyl, Ethyl, Propyl, Butyl, Trifluormethyl, Trifluormethoxy oder Difluormethoxy.

15. Zusammensetzung nach einem der Ansprüche 1 - 14 ausgewählt aus der Gruppe der folgenden Verbindungen:
1-Anilino-3-phenyl-pyrrol-2,5-dion,
1-Anilino-3-p-tolyl-pyrrol-2,5-dion,
1-(N-Methyl-anilino)-3-p-tolyl-pyrrol-2,5-dion,
1-Anilino-3-(3-chloro-phenyl)-pyrrol-2,5-dion,
1-Anilino-3-(4-chloro-phenyl)-pyrrol-2,5-dion,
1-Anilino-3-(4-bromo-phenyl)-pyrrol-2,5-dion,
3-(4-Chloro-phenyl)-1-(N-methyl-anilino)-pyrrol-2,5-dion,
1-(4-Chloro-anilino)-3-p-tolyl-pyrrol-2,5-dion,
3-(4-Bromo-phenyl)-1-(4-methyl-anilino)-pyrrol-2,5-dion,
1-(4-Chloro-anilino)-3-(4-chloro-phenyl)-pyrrol-2,5-dion,
1-Anilino-3-(3,4-dichlor-phenyl)-pyrrol-2,5-dion,
3-(4-Bromo-phenyl)-1-(4-methoxy-anilino)-pyrrol-2,5-dion,
3-(4-Chlor-phenyl)-1-(3,4-dichlor-anilino)-pyrrol-2,5-dion,
3-(4-Methoxy-phenyl)-1-(N-methyl-anilino)-pyrrol-2,5-dion,
1-Anilino-3-(4-methoxy-phenyl)-pyrrol-2,5-dion,
1-(4-Chloro-anilino)-3-(4-methoxy-phenyl)-pyrrol-2,5-dion,
1-(4-Methoxy-anilino)-3-(4-methoxy-phenyl)-pyrrol-2,5-dion,
1-(2-Methoxy-anilino)-3-(4-methoxy-phenyl)-pyrrol-2,5-dion,
1-(4-Fluoro-anilino)-3-(4-chloro-phenyl)-pyrrol-2,5-dion,
3-(4-Chlor-phenyl)-1-(4-methyl-anilino)-pyrrol-2,5-dion,
1-(4-Methyl-anilino)-3-phenyl-pyrrol-2,5-dion,
3-(4-Chlor-phenyl)-1-(2,4-dichlor-anilino)-pyrrol-2,5-dion,
1-(2, 4-Dichlor-anilino)-3-phenyl-pyrrol-2,5-dion,
1-(2, 4-Dichlor-anilino)-3-(4-methoxy-phenyl)-pyrrol-2,5-dion.

16. Verbindungen der Formel II 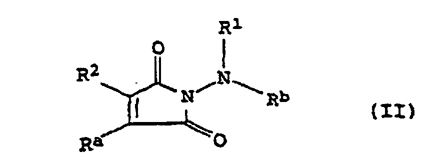 wobei die Reste R¹, R², R^{a} und R^{b} folgende Bedeutungen haben:
R¹ Formyl;
R² Wasserstoff;
R^{a} Phenyl, das ein- oder mehrfach, vorzugsweise ein- oder zweifach substituiert sein kann durch Halogen, oder durch eine Phenylgruppe, die ihrerseits ebenfalls durch Halogen oder C₁-C₄-Alkyl substituiert sein kann;
R^{b} Phenyl, das ein- oder mehrfach, vorzugsweise ein- bis vierfach, substituiert sein kann durch Halogen, C₁-C₆-Alkyl, C₁-C₄-Halogenalkyl, C₁-C₄-Halogenalkoxy.

17. Verwendung von fungiziden Mitteln nach einem der Ansprüche 1 - 15 oder von Verbindungen der Formel II nach Anspruch 16 zur Bekämpfung von Schadpilzen in der Landwirtschaft.

18. Verfahren zur Bekämpfung von Schadpilzen, **dadurch gekennzeichnet, daß** man die Schadpilze, deren Lebensraum oder die von ihnen freizuhaltenden Pflanzen, Flächen, Materialien oder Räume mit einer fungizid wirksamen Menge eines Mittels gemäß einem der Ansprüche 1 - 15 oder von Verbindungen der Formel II nach Anspruch 16 behandelt.

19. Agrochemische Kombinationspräparate enthaltend als Wirkstoffe Verbindungen der Formel I gemäß den Ansprüchen 1 - 15 oder von Verbindungen der Formel II nach Anspruch 16, sowie mindestens einen weiteren fungiziden Wirkstoff.

20. Verwendung von fungiziden Wirkstoffen in Kombination mit mindestens einem Wirkstoff der Formel I gemäß einem der Ansprüche 1 - 15 oder von Verbindungen der Formel II nach Anspruch 16 zur Bekämpfung von Schadpilzen in der Landwirtschaft.

## Claims

1. An agrochemical composition having fungicidal action, comprising as active compounds compounds of the formula I 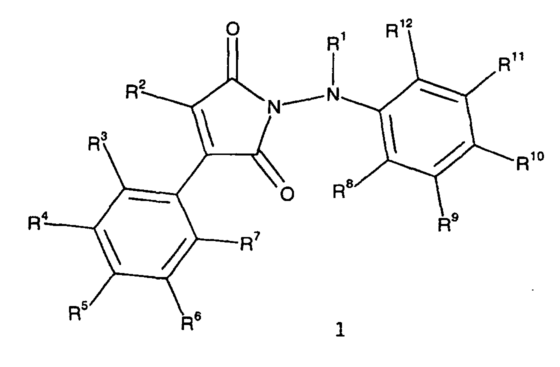 where:
R¹ is hydrogen, C₁-C₆-alkyl, C₁-C₆-alkylcarbonyl, formyl or C₁-C₆-haloalkylcarbonyl;
R² is hydrogen, C₁-C₆-alkyl or C₁-C₆-haloalkyl;
R³ - R¹² are hydrogen, halogen, C₁-C₈-cycloalkyl, C₁-C₆-alkyl, C₁-C₆-haloalkyl, C₁-C₆-alkoxy, C₁-C₆-haloalkoxy, C₁-C₆-alkylsulfonyl, C₁-C₆-haloalkylsulfonyl, formyl, C₁-C₆-alkylcarbaonyl, cyano, C₁-C₆-alkylthio or phenyl, which may be unsubstituted or substituted by halogen atoms, C₁-C₆-alkyl or C₁-C₆-haloalkyl groups, and their agriculturally useful salts.

2. A composition as claimed in claim 1, where R¹ is hydrogen, C₁-C₆-alkyl or formyl.

3. A composition as claimed in claim 2, where R¹ is hydrogen or methyl.

4. A composition as claimed in any of claims 1-3, where R² is hydrogen, C₁-C₆-alkyl or trifluoromethyl.

5. A composition as claimed in claim 4, where R² is hydrogen.

6. A composition as claimed in any of claims 1 - 5, where one or more of the radicals R³-R¹² are as defined below: fluorine, chlorine, methyl, ethyl, propyl, butyl, trifluoromethyl, difluoromethyl, trifluoromethoxy, difluoromethoxy, methoxy, methylthio, cyano and the remaining radicals R³-R¹² are hydrogen.

7. A composition as claimed in claim 6, where four to nine of the radicals R³-R¹² are hydrogen.

8. A composition as claimed in any of claims 1 - 7, where two to five of the radicals R⁸-R¹² are hydrogen.

9. A composition as claimed in claim 8, where three or four of the radicals R⁸-R¹² are hydrogen.

10. A composition as claimed in any of claims 1 - 9, where two to five of the radicals R³-R⁷ are hydrogen.

11. A composition as claimed in claim 10, where three or four of the radicals R³-R⁷ are hydrogen.

12. A composition as claimed in any of claims 1 - 11, where at least two of the radicals R⁸-R¹² and at least two of the radicals R³-R⁷ are hydrogen.

13. A composition as claimed in any of claims 1 - 12, where one, two or three of the radicals R³-R¹² are halogen, C₁-C₆-alkyl, C₁-C₆-haloalkyl, C₁-C₆-alkoxy or C₁-C₆-haloalkoxy.

14. A composition as claimed in claim 13, where the radicals R³-R¹² are selected from the group consisting of fluorine, chlorine, bromine, iodine, methyl, ethyl, propyl, butyl, trifluoromethyl, trifluoromethoxy and difluoromethoxy.

15. A composition as claimed in any of claims 1 - 14, selected from the group of the following compounds:
1-anilino-3-phenylpyrrole-2,5-dione,
1-anilino-3-p-tolylpyrrole-2,5-dione,
1-(N-methylanilino)-3-p-tolylpyrrole-2,5-dione,
1-anilino-3-(3-chlorophenyl)pyrrole-2,5-dione,
1-anilino-3-(4-chlorophenyl)pyrrole-2,5-dione,
1-anilino-3-(4-bromophenyl)pyrrole-2,5-dione,
3-(4-chlorophenyl)-1-(N-methylanilino)pyrrole-2,5-dione,
1-(4-chloroanilino)-3-p-tolylpyrrole-2,5-dione,
3-(4-bromophenyl)-1-(4-methylanilino)pyrrole-2,5-dione,
1-(4-chloroanilino)-3-(4-chlorophenyl)pyrrole-2,5-dione,
1-anilino-3-(3,4-dichlorophenyl)pyrrole-2,5-dione,
3-(4-bromophenyl)-1-(4-methoxyanilino)pyrrole-2,5-dione,
3-(4-chlorophenyl)-1-(3,4-dichloroanilino)pyrrole-2,5-dione,
3-(4-methoxyphenyl)-1-(N-methylanilino)pyrrole-2,5-dione,
1-anilino-3-(4-methoxyphenyl)pyrrole-2,5-dione,
1-(4-chloroanilino)-3-(4-methoxyphenyl)pyrrole-2,5-dione,
1-(4-methoxyanilino)-3-(4-methoxyphenyl)pyrrole-2,5-dione,
1-(2-methoxyanilino)-3-(4-methoxyphenyl)pyrrole-2,5-dione,
1-(4-fluoroanilino)-3-(4-chlorophenyl)pyrrole-2,5-dione,
3-(4-chlorophenyl)-1-(4-methylanilino)pyrrole-2,5-dione,
1-(4-methylanilino)-3-phenylpyrrole-2,5-dione,
3-(4-chlorophenyl)-1-(2,4-dichloroanilino)pyrrole-2,5-dione,
1-(2,4-dichloroanilino)-3-phenylpyrrole-2,5-dione,
1-(2,4-dichloroanilino)-3-(4-methoxyphenyl)pyrrole-2,5-dione.

16. A compound of the formula II 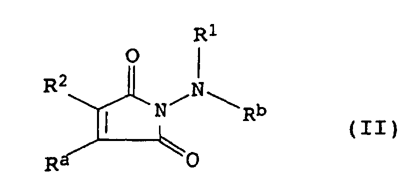 in which the radicals R¹, R², R^{a} and R^{b} are as defined below:
R¹ is formyl;
R² is hydrogen;
R^{a} is phenyl which may be mono- or polysubstituted, preferably mono- or disubstituted, by halogen, or by a phenyl group which for its part may likewise be substituted by halogen or C₁-C₄-alkyl;
R^{b} is phenyl which may be mono- or polysubstituted, preferably mono- to tetrasubstituted, by halogen, C₁-C₆-alkyl, C₁-C₄-haloalkyl, C₁-C₄-haloalkoxy.

17. The use of fungicidal compositions as claimed in any of claims 1 - 15 or of compounds of the formula II as claimed in claim 16 for controlling harmful fungi in agriculture.

18. A method for controlling harmful fungi, wherein the harmful fungi, their habitat or the plants, areas, materials or spaces to be kept free from them are treated with a fungicidally effective amount of a composition as claimed in any of claims 1 - 15 or of compounds of the formula II as claimed in claim 16.

19. An agrochemical combination preparation comprising, as active compounds, compounds of the formula I as claimed in any of claims 1 - 15 or compounds of the formula II as claimed in claim 16, and at least one further fungicidally active compound.

20. The use of fungicidally active compounds in combination with at least one active compound of the formula I as claimed in any of claims 1 - 15 or of compounds of the formula II as claimed in claim 16 for controlling harmful fungi in agriculture.

## Revendications

1. Composition agrochimique à action fongicide contenant comme substances actives des composés de formule I 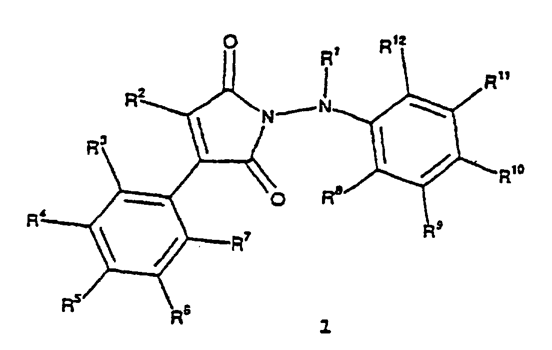 où les restes ont les significations suivantes:
R¹ hydrogène, alkyle en C₁-C₆, alkylcarbonyle en C₁-C₆, formyle ou halogénoalkyl(C₁-C₆) carbonyle;
R² hydrogène, alkyle en C₁-C₆ ou halogénoalkyle C₁-C₆;
R³-R¹² hydrogène, halogène, cycloalkyle en C₁-C₆, alkyle en C₁-C₆, halogénoalkyle en C₁-C₆, alcoxy en C₁-C₆, halogénoalcoxy en C₁-C₆, alkylsulfonyle en C₁-C₆, halogénoalkylsulfonyle en C₁-C₆, formyle, alkyl(C₁-C₆) carbonyle, cyano, alkylthio en C₁-C₆ ou phényle, qui peut être éventuellement substitué par des atomes d'halogène, des groupes alkyle en C₁-C₆ ou des groupes halogénoalkyle en C₁-C₆, ainsi que leurs sels utilisables en agriculture.

2. Composition selon la revendication 1, dans laquelle R¹ est l'hydrogène, un alkyle en C₁-C₆ ou le formyle.

3. Composition selon la revendication 2, dans laquelle R¹ désigne l'hydrogène ou le méthyle.

4. Composition selon l'une des revendications 1 à 3, dans laquelle R² désigne l'hydrogène, un alkyle en C₁-C₆ ou le trifluorométhyle.

5. Composition selon la revendication 4, dans laquelle R² désigne l'hydrogène.

6. Composition selon l'une des revendications 1 à 5, dans laquelle un ou plusieurs des restes R³-R¹² ont les significations suivantes: fluor, chlore, méthyle, éthyle, propyle, butyle, trifluorométhyle, difluorométhyle, trifluorométhoxy, difluorométhoxy, méthoxy, méthylthio, cyano, et les autres restes R³-R¹² désignent l'hydrogène.

7. Composition selon la revendication 6, dans laquelle quatre à neuf des restes R³-R¹² désignent l'hydrogène.

8. Composition selon l'une des revendications 1 à 7, dans laquelle deux à cinq des restes R⁸-R¹² désignent l'hydrogène.

9. Composition selon la revendication 8, dans laquelle trois ou quatre des restes R⁸-R¹² désignent l'hydrogène.

10. Composition selon l'une des revendications 1 à 9, dans laquelle deux à cinq des restes R³-R⁷ désignent l'hydrogène.

11. Composition selon la revendication 10, dans laquelle trois ou quatre des restes R³-R⁷ désignent l'hydrogène.

12. Composition selon l'une des revendications 1 à 11, dans laquelle au moins deux des restes R⁸-R¹² ainsi qu'au moins deux des restes R³-R⁷ désignent l'hydrogène.

13. Composition selon l'une des revendications 1 à 12, dans laquelle un, deux ou trois des restes R³-R¹² désignent un halogène, un alkyle en C₁-C₆, un halogénoalkyle en C₁-C₆, un alcoxy en C₁-C₆ ou un halogénoalcoxy en C₁-C₆.

14. Composition selon la revendication 13, dans laquelle les restes R³-R¹² sont choisis parmi le fluor, le chlore, le brome, l'iode, le méthyle, l'éthyle, le propyle, le butyle, le trifluoro-méthyle, le trifluorométhoxy ou le difluorométhoxy.

15. Composition selon l'une des revendications 1 à 14, choisie dans le groupe des composés suivants:
1-anilino-3-phényl-pyrrole-2,5-dione,
1-anilino-3-p-tolyl-pyrrole-2,5-dione,
1-(N-méthyl-anilino)-3-p-tolyl-pyrrole-2,5-dione,
1-anilino-3-(3-chloro-phényl)-pyrrole-2,5-dione,
1-anilino-3-(4-chloro-phényl)-pyrrole-2,5-dione,
1-anilino-3-(4-bromo-phényl)-pyrrole-2,5-dione,
3-(4-chloro-phényl)-1-(N-méthyl-anilino)-pyrrole-2,5-dione,
1-(4-chloro-anilino)-3-p-tolyl-pyrrole-2,5-dione,
3-(4-bromo-phényl)-1-(4- méthyl-anilino)-pyrrole-2,5-dione,
1-(4-chloro-anilino)-3-(4-chloro-phényl)-pyrrole-2,5-dione,
1-anilino-3-(3,4-dichloro-phényl)-pyrrole-2,5-dione,
3-(4-bromo-phényl)-1-(4-méthoxy-anilino)-pyrrole-2,5-dione,
3-(4-chloro-phényl)-1-(3,4-dichloro-anilino)-pyrrole-2,5-dione
3-(4-méthoxy-phényl)-1-(N-méthyl-anilino)-pyrrole-2,5-dione,
1-anilino-3-(4-méthoxy-phényl)-pyrrole-2,5-dione,
1-(4-chloro-anilino)-3-(4-méthoxy-phényl)-pyrrole-2,5-dione,
1-(4-méthoxy-anilino)-3-(4-méthoxy-phényl)-pyrrole-2,5-dione,
1-(2-méthoxy-anilino)-3-(4-méthoxy-phényl)-pyrrole-2,5-dione,
1-(4-fluoro-anilino)-3-(4-chloro-phényl)-pyrrole-2,5-dione,
3-(4-chloro-phényl)-1-(4-méthyl-anilino)-pyrrole-2,5-dione,
1-(4-méthyl-anilino)-3-phényl-pyrrole-2,5-dione,
3-(4-chloro-phényl)-1-(2,4-dichloro-anilino)-pyrrole-2,5-dione,
1-(2,4-dichloro-anilino)-3-phényl-pyrrole-2,5-dione,
1-(2,4-dichloro-anilino)-3-(4-méthoxy-phényl)-pyrrole-2,5-dione,

16. Composés de formule II 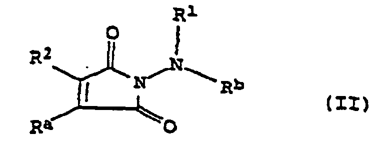 où les restes R¹, R², R^{a} et R^{b} ont les significations suivantes:
R¹ formyle;
R² hydrogène;
R^{a} phényle, qui peut être substitué une ou plusieurs fois, de préférence une ou deux fois, par des halogènes ou par un groupe phényle qui peut également à son tour être substitué par des halogènes ou un groupe alkyle en C₁-C₄;
R^{b} phényle, qui peut être substitué une ou plusieurs fois, de préférence une à quatre fois, par des halogènes, un alkyle en C₁-C₆, un halogénoalkyle en C₁-C₄, un halogénoalcoxy en C₁-C₄.

17. Utilisation des produits fongicides selon l'une des revendications 1 - 15, ou de composés de formule II selon la revendication 16 pour la lutte contre les champignons nuisibles en agriculture.

18. Procédé pour la lutte contre les champignons nuisibles, **caractérisé par le fait qu'**on traite les champignons nuisibles, leur biotope ou les plantes, surfaces, matériels ou espaces qui doivent en être exemptés avec une quantité à efficacité fongicide d'un produit selon l'une des revendications 1 à 15 ou de composés de formule II selon la revendication 16.

19. Préparation sous forme de combinaison agrochimique contenant comme substances actives des composés de formule I selon les revendications 1 à 15 ou des composés de formule II selon la revendication 16, ainsi qu'une autre substance à activité fongicide.

20. Utilisation de substances à activité fongicide en combinaison avec au moins une substance active de formule I selon l'une des revendications 1 à 15 ou des composés de formule II selon la revendication 16 pour la lutte contre les champignons nuisibles en agriculture.
